(19) Europäisches Patentamt
European Patent Office
Office européen des brevets

(11) EP 4 341 321 B1

# (12) EUROPEAN PATENT SPECIFICATION

(45) Date of publication and mention of the grant of the patent:
**05.03.2025 Bulletin 2025/10**

(21) Application number: **22728634.1**

(22) Date of filing: **23.05.2022**

(51) International Patent Classification (IPC):
***C08G 18/42*** (2006.01) ***C08L 67/04*** (2006.01)
***A61L 27/36*** (2006.01) ***A61L 27/58*** (2006.01)
***B33Y 10/00*** (2015.01) ***B33Y 70/00*** (2020.01)
***B33Y 80/00*** (2015.01)

(52) Cooperative Patent Classification (CPC):
**B33Y 10/00; A61L 27/3645; A61L 27/365; A61L 27/3654; A61L 27/58; B33Y 70/00; B33Y 80/00; C08F 290/061; C08G 18/227; C08G 18/4277; C08G 18/755; C08G 18/8166; C08G 18/8175; C08G 18/8191; C08G 18/835;**
(Cont.)

(86) International application number:
**PCT/EP2022/063904**

(87) International publication number:
**WO 2022/243567 (24.11.2022 Gazette 2022/47)**

## (54) CROSSLINKED BIORESORBABLE POLYESTER NETWORKS

VERNETZTE BIORESORBIERBARE POLYESTERNETZWERKE

RÉSEAUX DE POLYESTER BIORÉSORBABLES RÉTICULÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.05.2021 EP 21175197**

(43) Date of publication of application:
**27.03.2024 Bulletin 2024/13**

(73) Proprietor: **Universiteit Gent**
**9000 Gent (BE)**

(72) Inventors:
- **THIJSSEN, Quinten**
  **9120 Beveren (BE)**
- **VAN VLIERBERGHE, Sandra**
  **9100 Sint-Niklaas (BE)**

(74) Representative: **Arnold & Siedsma**
**Bezuidenhoutseweg 57**
**2594 AC The Hague (NL)**

(56) References cited:
**WO-A1-2017/005613 WO-A1-2020/094621**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**C08G 63/6852; C08G 63/912;** C08G 2230/00

## Description

FIELD OF THE INVENTION

The present invention relates to the field of bioresorbable crosslinked materials. More specifically, the present invention pertains to a combination comprising a polyester having a crystalline backbone and at least one end-capping group attached to a first crosslinkable group and a compound comprising a second crosslinkable group, a crosslinked polyester wherein said combination forms a crosslinked network, a process of manufacturing thereof and the use of said crosslinked polyester.

BACKGROUND TO THE INVENTION

Bioresorbable synthetic polymers such as poly(ε-caprolactone) (PCL), poly(lactic acid) and poly(lactic-co-glycolic acid) (PLGA) have been widely studied in the context of regenerative medicine (RM) and tissue engineering (TE). Due to their tailorable degradability and mechanical properties combined with excellent biocompatibility, these polymers are the ideal candidates for TE. Furthermore, their suitability for 3D-printing in order to manufacture patient-specific implants (PSI) has attracted attention in recent years as the use of PSIs is reported to reduce operation times, operative tissue damage and post-operative infections as a result of the excellent fit with the anatomical structure of the patient.

In this context, light-based additive manufacturing techniques such as two-photon polymerization (2PP), stereolithography (SLA) and digital light processing (DLP) offer distinct benefits in terms of manufacturing precision and throughput compared to more conventional extrusion-based techniques. However, despite these advantages, the application of light-based additive manufacturing techniques in the context of regenerative tissue engineering remains limited due to the lack of photopolymerizable bioresorbable materials with appropriate mechanical properties and photo-reactivity.

The established approach in the state-of-the-art to obtain photo-crosslinkable bioresorbable materials is via the chemical functionalization of a polyester in order to incorporate photo-crosslinkable acrylate end-groups. Currently, various biodegradable polymers such as PCL, PLA and their copolymers have been functionalized via this approach. However, the acrylate-terminated polyesters, obtained via this strategy, result in brittle networks (characterized by a low elongation at break and low ultimate strength).

Tian, G. *et al.,* 2019 have described acrylate terminated PCL comprising shape-memory networks. Nevertheless, the acrylated polyesters in accordance with Tian, G. *et al.,* 2019, have the drawback of having limited mechanical properties, not optimal for their use in biomedical applications, e.g. reaching a maximum strength of 11.6 MPa.

WO 2020/094621 A1 discloses a combination comprising acrylate end-capped urethane-based polymers, which are in particular characterized by the presence of at least 3 acrylate end-groups, thereby making them particularly suitable for further processing using multiple kinds of technologies such as 2-photon polymerization, stereolithography, electrospinning, film casting, porogen leaching, extrusion based 3D-printing, spray drying, cryogenic treatment, coatings, crosslinkable micelles, spincoating, and electrospraying. The acrylate end-capped urethane-based polymers may have a polyester backbone. The combination further comprises a first crosslinkable group and a second crosslinkable group, and is used to manufacture biomedical devices such as implants, having improved mechanical properties.

Therefore, there is a need for bioresorbable crosslinked materials having improved mechanical and curing characteristics. The present invention provides for bioresorbable crosslinked materials, combinations providing for said materials and a process of manufacturing thereof overcoming the drawbacks in the prior art.

SUMMARY OF THE INVENTION

In a first aspect, the present invention relates to a combination comprising:

- at least a polyester having a crystalline backbone and at least one end-capping group, the end-capping group being a group connected to a terminal end of said polyester chain, wherein the end-capping group comprises any one of an ester, urethane, thiocarbamate or urea moiety connected either directly or by means of a spacer (therefore indirectly) to a first crosslinkable group, meaning a group adapted to provide a crosslinked network, and being part of said polyester;
- at least a compound comprising a second crosslinkable group, and therefore being part of said compound;

wherein the first and second crosslinkable groups are selected from either:

- a thiol comprising group; or

- an -ene group of formula (I),

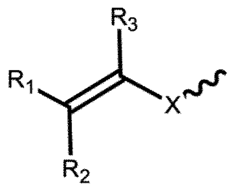

(I)

wherein

◦ $R_1$, $R_2$, $R_3$ are independently selected from: H, alkyl, O, N, halogen, S or at least one of $R_1$, $R_2$, $R_3$ together with the group at position X form a cyclic or heterocyclic structure;
◦ X is a group selected from: alkyl, O, N, S, (C=O)N, (C=O)alkyl, O-alkyl, N-alkyl, S-alkyl, and

wherein at least one group of the first and second crosslinkable groups is the thiol comprising group and at least one group of the first and second crosslinkable groups is the -ene group of formula (I), and

wherein the polyester and the compound comprise at least 2 of a first, respectively second crosslinkable group, and the sum of said first, respectively second crosslinkable groups, is at least 5 (for example 2 thiol comprising groups on the compound and 3 -ene groups of formula (I) on the polyester, or vice-versa).

In accordance with an embodiment of the present invention in the combination, the polyester is of formula (B)

$$((A_m)_y - Y_m - Z_m)_n \text{ - backbone (B)}$$

wherein:

- $((A_m)_y - Y_m - Z_m)_n$ represents at least one end-capping group, and
- $A_m$ represents the first crosslinkable group;
- $Y_m$ is selected from the list comprising: the direct bond or the spacer;
- $Z_m$ represents the moiety selected from ester, urethane, thiocarbamate or urea, connected to said backbone (the polyester backbone), directly or indirectly; and

wherein n is integer and defines the number of arms/branches connected to the polyester backbone, with $n \geq 1$, and wherein y is an integer and defines the number of first crosslinkable groups, and m is an enumerator for each arm/branch.

In accordance with a further embodiment of the present invention, in the combination, the molar ratio of the first crosslinkable group to the second crosslinkable group is from 0.75 to 1.5, preferably from 0.8 to 1.2, e.g. the molar ratio of the -ene group of formula (I) to the thiol comprising group, is from 0.75 to 1.5, preferably from 0.8 to 1.2.

In accordance with a further embodiment of the present invention in the combination, the polyester comprises the urethane moiety and the first crosslinkable group is the -ene group moiety of formula (I).

In accordance with a further embodiment of the present invention in the combination, the polyester comprises at least 2 end-capping groups comprising each at least one -ene group moiety of formula (I).

In accordance with a further embodiment of the present invention in the combination, the backbone has a crystallinity in the range from about 10% to 80%, preferably from 20% to 70%, more preferably from 30% to 60%.

In accordance with a further embodiment of the present invention in the combination the backbone has a molar mass in the range from about 500 $g.mol^{-1}$ to 20000 $g.mol^{-1}$, preferably from 2000 $g.mol^{-1}$ to 10000 $g.mol^{-1}$.

In accordance with a further embodiment of the present invention in the combination, the polyester has a geometry selected from: linear, star-shaped, preferably star-shaped.

In accordance with a further embodiment of the present invention in the combination, the polyester is selected from poly($\varepsilon$-caprolactone) (PCL), poly(lactic acid) (PLA), poly(lactic-co-glycolic acid) (PLGA), polyglycolic acid (PGA) and copolymers thereof, preferably PLA and/or PCL.

In accordance with a further embodiment of the present invention in the combination the -ene group of formula (I) is selected from: alkene, norbornene, allyl ether, vinyl ether, maleimide, preferably alkene, norbornene, allyl ether, vinyl ether.

In accordance with a further embodiment of the present invention in the combination, the compound comprising the second crosslinkable group is selected from: 1,2-Ethanedithiol, 1,3-Propanedithiol, 1,4-Butanedithiol, 2,2'-Thiodiethanethiol, 2,2'-(Ethylenedioxy)diethanethiol, Trimethylolpropane tris(3-mercaptopropionate), Pentaerythritol tetrakis(3-mercaptopropionate), 2-hydroxymethyl-2-methyl-1,3-propanediol tris-(3-mercaptopropionate) and combinations thereof.

In a second aspect, the present invention relates to a crosslinked polyester comprising a combination as defined in accordance with the present invention wherein the polyester and the compound form a crosslinked network.

In a third aspect, the present invention relates to a process to produce a crosslinked polyester according to the present invention, comprising the steps of:

a) providing a combination as defined in accordance with the present invention,
b) crosslinking the combination provided at step a).

In accordance with an embodiment of the present invention, in the process step a) further comprises providing an initiator to the combination, such as a photoinitiator, a thermal initiator or a redox initiator.

In a third aspect, the present invention relates to a use of a combination as defined in accordance with the present invention and/or a crosslinked polyester as defined in accordance with the present invention, in additive manufacturing as a bioink, bioengineering, biomedical applications, such as implants, preferably biodegradable implants, such as bone implants, cartilage implants.

## BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

**Figure 1,** also abbreviated as **Fig. 1**, illustrates the two-step synthesis protocol of the acrylate-(AUP PCL), alkene-(EUP PCL) and alkyne- (YUP PCL) terminated PCLs. Wherein alkene terminated PCLs (EUP PCLs) are polyesters in accordance with the present invention.

**Figure 2,** also abbreviated as **Fig. 2**, illustrates a schematic representation of the different topologies of the PCL-based networks, stemmed from the involved crosslinking chemistries; A. Acrylate crosslinking (AUP PCL); B. Acrylate-thiol binary crosslinking (AUP PCL + PETA 4SH); C. Thiol-ene crosslinking (EUP PCL + PETA 4SH); D. Thiol-yne crosslinking (YUP PCL + PETA 4SH). Wherein C represents a crosslinked polyester in accordance with the present invention, wherein an alkene terminated PCL (EUP PCL) is crosslinked with pentaerythritol tetrakis(3-mercaptopropionate) (PETA 4SH).

**Figure 3,** also abbreviated as **Fig. 3**, illustrates the photo-rheology of the different PCL-based networks tested, including the one of crosslinked polyester in accordance with the present invention obtained from the combination EUP PCL + PETA 4SH.

**Figure 4,** also abbreviated as **Fig. 4**, illustrates DSC thermograms of the PCL-based networks tested, including the one of crosslinked polyester in accordance with the present invention obtained from the combination EUP PCL + PETA 4SH.

**Figure 5,** also abbreviated as **Fig. 5**, illustrates DSC thermograms showing the initial crystallinity (after storage at room temperature for one week) of the four PCL-based networks, including the one of crosslinked polyester in accordance with the present invention obtained from the combination EUP PCL + PETA 4SH.

**Figure 6,** also abbreviated as **Fig. 6**, illustrates stress-strain plots obtained via tensile testing for the different crosslinked networks tested, including the one of crosslinked polyester according to the present invention obtained from the combination EUP PCL + PETA 4SH.

**Figure 7,** also abbreviated as **Fig. 7**, illustrates a neutral red uptake experiment according to ISO standard 10993 in order to investigate the biocompatibility of the four PCL-based networks, including the one of crosslinked polyester according to the present invention obtained from the combination EUP PCL + PETA 4SH.

**Figure 8,** also abbreviated as **Fig. 8**, illustrates the synthesis of alkene-terminated PCL with 2 urethane-functionalities and a central PCL-segment.

**Figure 9,** also abbreviated as **Fig. 9**, illustrates the influence of the molar mass of the central PCL-segment on the mechanical properties determined via tensile testing of the compounds obtained according to the scheme of **Fig. 8.**

**Figure 10,** also abbreviated as **Fig. 10**, illustrates the influence of the architecture of the central PCL-segment on the mechanical properties determined via tensile testing of the compounds obtained according to the scheme of **Fig. 8.**

**Figure 11,** also abbreviated as **Fig. 11,** illustrates the influence of the functionality of the thiol-crosslinker on the mechanical properties determined via tensile testing of the compounds obtained according to the scheme of **Fig. 8.**

**Figure 12,** also abbreviated as **Fig. 12,** illustrates the comparison of acrylate cross-linking and thiol-ene cross-linking for photo-crosslinked PCL networks with identical molar masses.

## DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous. When describing the compounds of the invention, the terms used are to be construed in accordance with the following definitions, unless a context dictates otherwise. The term "about" or "approximately" as used herein when referring to a measurable value such as a parameter, an amount, a temporal duration, and the like, is meant to encompass variations of +/- 10 % or less, preferably +/- 5 % or less, more preferably +/- 1 % or less, and still more preferably +/- 0.1 % or less of and from the specified value, insofar such variations are appropriate to perform in the disclosed invention. It is to be understood that the value to which the modifier "about" or "approximately" refers is itself also specifically, and preferably, disclosed.

In a first aspect, the present invention relates to a combination comprising:

- at least a polyester having a crystalline backbone and at least one end-capping group, wherein the end-capping group comprises any one of an ester, urethane, thiocarbamate or urea moiety connected either directly or by means of a spacer to a first crosslinkable group;
- at least a compound comprising a second crosslinkable group, wherein the first and second crosslinkable group are selected from either:
  - a thiol comprising group; or
  - an -ene group of formula (I),

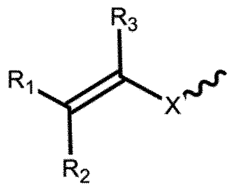

(I)

wherein

◦ $R_1$, $R_2$, $R_3$ are independently selected from: H, alkyl, O, N, halogen, S or at least one of $R_1$, $R_2$, $R_3$ together with the group at position X form a cyclic or heterocyclic structure;
o X is a group selected from: alkyl, O, N, S, (C=O)N, (C=O)alkyl, O-alkyl, N-alkyl, S-alkyl, and

wherein at least one group of the first and second crosslinkable groups is the thiol comprising group and at least one group of the first and second crosslinkable groups is the -ene group of formula (I), and

wherein the polyester and the compound comprise at least 2 of a first, respectively second crosslinkable groups, and the sum of said first, respectively second crosslinkable groups, is at least 5.

In accordance with the present invention, by means of the term "combination", reference is made to the product obtained from combining two or more compounds together. In accordance with the present invention, by means of the term "compound", reference is made to a chemical compound, in other words, a molecule, of any size e.g. macromolecule such as a polymer or a small molecule. In the present case, the two products combined together are said polyester and said compound defined in accordance with the present invention.

The term "alkyl" by itself or as part of another substituent refers to a fully saturated hydrocarbon of Formula $C_xH_{2x+1}$ wherein x is a number greater than or equal to 1. Generally, alkyl groups of this invention comprise from 1 to 20 carbon atoms. Alkyl groups may be linear or branched and may be substituted as indicated herein. When a subscript is used herein following a carbon atom, the subscript refers to the number of carbon atoms that the named group may contain. Thus, for example, $C_{1-4}$alkyl means an alkyl of one to four carbon atoms. Examples of alkyl groups are methyl, ethyl, n-propyl, i-propyl, butyl, and its isomers (e.g. n-butyl, i-butyl and t-butyl); pentyl and its isomers, hexyl and its isomers, heptyl and its isomers, octyl and its isomers, nonyl and its isomers; decyl and its isomers. $C_1$-$C_6$ alkyl includes all linear, branched, or cyclic alkyl groups with between 1 and 6 carbon atoms, and thus includes methyl, ethyl, n-propyl, i-propyl,

butyl and its isomers (e.g. n-butyl, i-butyl and t-butyl); pentyl and its isomers, hexyl and its isomers, cyclopentyl, 2-, 3-, or 4-methylcyclopentyl, cyclopentylmethylene, and cyclohexyl. The term "heterocyclic" as used herein by itself or as part of another group referto non-aromatic, fully saturated or partially unsaturated cyclic groups (for example, 3 to 13 member monocyclic, 7 to 17 member bicyclic, or 10 to 20 member tricyclic ring systems, or containing a total of 3 to 10 ring atoms) which have at least one heteroatom in at least one carbon atom-containing ring. Each ring of the heterocyclic group containing a heteroatom may have 1, 2, 3 or 4 heteroatoms selected from nitrogen atoms, oxygen atoms and/or sulfur atoms, where the nitrogen and sulfur heteroatoms may optionally be oxidized and the nitrogen heteroatoms may optionally be quaternized. The heterocyclic group may be attached at any heteroatom or carbon atom of the ring or ring system, where valence allows. The rings of multi-ring heterocycles may be fused, bridged and/or joined through one or more spiro atoms.

Exemplary heterocyclic groups include piperidinyl, azetidinyl, imidazolinyl, imidazolidinyl, isoxazolinyl, oxazolidinyl, isoxazolidinyl, thiazolidinyl, isothiazolidinyl, piperidyl, succinimidyl, 3H-indolyl, isoindolinyl, chromenyl, isochromanyl, xanthenyl, 2H-pyrrolyl, 1-pyrrolinyl, 2-pyrrolinyl, 3-pyrrolinyl, pyrrolidinyl, 4H-quinolizinyl, 4aH-carbazolyl, 2-oxopiperazinyl, piperazinyl, homopiperazinyl, 2-pyrazolinyl, 3-pyrazolinyl, pyranyl, dihydro-2H-pyranyl, 4H-pyranyl, 3,4-dihydro-2H-pyranyl, phthalazinyl, oxetanyl, thietanyl, 3-dioxolanyl, 1,3-dioxanyl, 2,5-dioximidazolidinyl, 2,2,4-piperidonyl, 2-oxopiperidinyl, 2-oxopyrrolodinyl, 2-oxoazepinyl, indolinyl, tetrahydropyranyl, tetrahydrofuranyl, tetrehydrothienyl, tetrahydroquinolinyl, tetrahydroisoquinolinyl, thiomorpholinyl, thiomorpholinyl sulfoxide, thiomorpholinyl sulfone, 1,3-dioxolanyl, 1,4-oxathianyl, 1,4-dithianyl, 1,3,5-trioxanyl, 6H-1,2,5-thiadiazinyl, 2H-1,5,2-dithiazinyl, 2H-oxocinyl, 1H-pyrrolizinyl, tetrahydro-1,1-dioxothienyl, N- formylpiperazinyl, and morpholinyl. The term "cyclic", "cyclic alkyl" or "cycloalkyl" as used herein by itself or as part of another substituent is a cyclic alkyl group, that is to say, a monovalent, saturated, or unsaturated hydrocarbyl group having 1, 2, or 3 cyclic structure. Cycloalkyl includes all saturated or partially saturated (containing 1 or 2 double bonds) hydrocarbon groups containing 1 to 3 rings, including monocyclic, bicyclic, or polycyclic alkyl groups. Cycloalkyl groups may comprise 3 or more carbon atoms in the ring and generally, according to this invention comprise from 3 to 15 atoms. The further rings of multi-ring cycloalkyls may be either fused, bridged and/or joined through one or more spiro atoms. Examples of cycloalkyl groups include but are not limited to cyclopropyl, cyclobutyl, cyclopentyl, cyclohexyl, cycloheptyl, cyclooctyl, cyclononyl, adamantanyl and cyclodecyl.

In accordance with the present invention, by means of the term "polyester", reference is made to a polymer comprising polymer units linked by ester groups. In accordance with the present invention, the polyester is provided with a crystalline backbone.

In accordance with the present invention, by means of the term "crystalline", reference is made to the property of a compound, such as a polymer, of comprising regions characterized by a degree of long-range order, and by means of the term "backbone", reference is made to a backbone chain of a polymer molecule, in this case the polyester. In other words, reference is made to the longest series of covalently bonded atoms that together create the continuous chain of the polyester. Therefore suitable polyesters to apply the present invention are polyesters provided with a crystalline backbone. Any polyester comprising a crystalline backbone is suitable to carry out the present invention. In accordance with the present invention, the crystallinity was measured via the melting enthalpy which was determined using differential scanning calorimetry (DSC). In the case of PCL crystallinity, the determined value by DSC is divided by 135 J/g (theoretical melting enthalpy of 100 % crystalline PCL, as reported in the following article of Nagata et al., 2009) and multiplied by 100 to determine the % crystallinity.

In accordance with an embodiment of the present invention in the combination the backbone has a crystallinity in the range from about 10% to 80%, preferably from 20% to 70%, more preferably from 30% to 60%.

In accordance with an embodiment of the present invention the polyester is selected from poly(ε-caprolactone) (PCL), poly(lactic acid) (PLA), poly(lactic-co-glycolic acid) (PLGA), polyglycolic acid (PGA) and copolymers thereof, preferably PLA and/or PCL.

PCL used in the context of the present invention can be PCL obtained using a variety of initiators, such as ethylene glycol, glycerol and pentaerythritol.

In accordance with an embodiment of the present invention in the combination the backbone of the polyester has a molar mass in the range from about 500 $g.mol^{-1}$ to 20000 $g.mol^{-1}$, preferably from 2000 $g.mol^{-1}$ to 10000 $g.mol^{-1}$. The molar mass or MM (units g/mole) of the backbone corresponds with the MM of the polyester prior to functionalization. The molar mass of the spacers and of the end-capping groups has to be added to the respective molar mass of the backbone in order to obtain the molar mass of the complete molecule.

In accordance with a further embodiment of the present invention in the combination the polyester has a geometry selected from: linear, star-shaped, preferably star-shaped. In other words, the polyester is provided with a backbone having a star-shaped backbone or a linear backbone. The geometry (e.g. linear and star-shaped) is determined by the initiator which is used during the polymerization of the polyester. A bifunctional initiator will lead to a linear polymer with two functional chain ends while a trifunctional initiator will lead to a star-shaped polymer with three functional chain ends.

Further, polyesters in accordance with the present invention further comprise at least one end-capping group,

wherein the end-capping group comprises any one of an ester, urethane, thiocarbamate or urea moiety. In accordance with the present invention, by means of the term "end-capping group", reference is made to a chemical group connected to a terminal end of a polymer chain. End-capping the polyester required by the present invention with anyone of ester, urethane, thiocarbamate or urea comprising moiety provides for improved workability of the crosslinked polyester, obtainable by crosslinking the polyester with a compound having a crosslinkable group in accordance with the present invention, especially when the end-capping group is urethane. Urethane (II), urea (III), and thiocarbamate (O-organyl (IV) and S-organyl (V)) and ester (VI) moieties can be represented as follows:

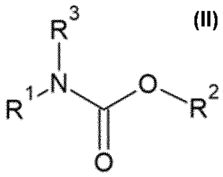
(II)

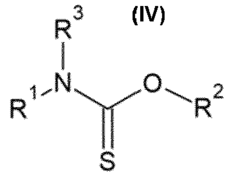
(IV)

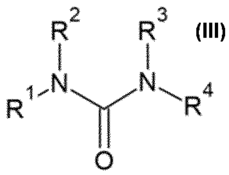
(III)

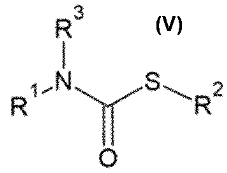
(V)

(VI)
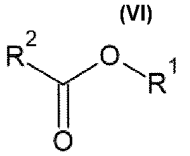

wherein $R^1$, $R^2$, $R^3$ and $R^4$ are independently selected from different atoms, such as C and H. It would be clear to the skilled in the art that the nature of groups $R^1$, $R^2$, $R^3$ and $R^4$ depends on the specific moiety represented. It would be clear to the skilled in the art that in formula (VI) above, $R^1$, $R^2$ are C, and in formulae (II), (IV), (V) $R^2$ is C and at least one of $R^1$, $R^3$ is C, and in formula (III) at least one of $R^1$, $R^2$, and at least one of $R^3$, $R^4$ is C. The end-capping group can comprise one or more of an ester, urethane, thiocarbamate or urea moiety. For example, the end-capping group in accordance with the present invention can e.g. comprise a single urethane moiety, two urethane moieties or more.

For example, polyesters according to the present invention comprising end-capping groups having two urethane moieties are shown in Fig. 1, wherein IPDI provides for the presence of 2 urethane moieties in each end-capping group. Further, polyesters according to the present invention can be obtained from PCL comprising backbones and allyl isocyanate, thereby providing for a single urethane moiety in each end capping group. Further polyesters according to the present invention can for example be obtained by reacting a polyester backbone such as PCL with e.g. 4-pentenoyl chloride, thereby providing end-capping groups each having an ester moiety.

In accordance with the present invention, the end-capping group is connected either directly or indirectly, such as by means of a spacer, to a first crosslinkable group. In accordance with the present invention, the compound comprises also a second crosslinkable group. The compound can be any compound allowing a second crosslinkable group to be attached thereon. It has to be understood that in accordance with the present invention, the first crosslinkable group is a crosslinkable group attached to the ester, urethane, carbamate or urea moiety, and is therefore part of the polyester, whilst the second crosslinkable group is comprised in the compound and is part thereof.

In accordance with the present invention, by means of the term "crosslinkable group", reference is made to a group provided to crosslink, thereby forming a covalent bond with another group it can react with, by means of a chemical reaction. The chemical reaction can be initiated with or without the intervention of another entity, such as UV light, heat, or another compound. More specifically, photoinitiators, thermal initiators and/or a redox initiators can be used to facilitate crosslinking. Examples of suitable photoinitiators are Eosin Y, diphenyl(2,4,6-trimethylbenzoyl)phosphine oxide (TPO), Ethyl (2,4,6-trimethylbenzoyl) phenyl phosphinate (TPO-L), Phenylbis(2,4,6-trimethylbenzoyl)phosphine oxide (BAPO), 4,4'-bis(dimethylamino)benzophenone and Irgacure 2959. The selection of the type of photoinitiator used depends the laser wavelength with which the network is crosslinked. Generally, photoinitiators in the UVA-visible light range (such as TPO, TPO-L, BAPO and Eosin Y) are preferred since these lower energy intensive wavelengths are less damaging for the materials. However, the type of initiator will generally not affect the final material properties. Suitable redox and/or thermal initiators are: 4,4'-Azobis(4-cyanovaleric acid), 1,1'-Azobis(cyclohexanecarbonitrile), 2,2'-Azobis(2-methylpropionamidine) dihydrochloride granular, 2,2'-Azobis(2-methylpropionitrile), Luperox®, Dicumyl peroxide, tert-Butyl hydroperoxide,

Cumene hydroperoxide, benzoylperoxide.

In accordance with the present invention, by means of the term "spacer", also known as "linker" reference is made to a molecule, preferably a flexible molecule, used to connect at least two molecules of interest together. In the present case, the spacer connects the end-capping group to the first crosslinkable group. Possible spacers for the present invention can be for example, and not limited to, caprolactone, lactide, glycolide, ethylene oxide, propylene oxide or aliphatic hydrocarbons such as methyl, ethyl, propyl, butyl, pentyl, hexyl.

In accordance with the present invention, the first and second crosslinkable group are selected from either a thiol comprising group or an -ene group of formula (I). The thiol comprising group and the -ene group are provided to react together thereby forming an C-S bond between the polyester and the compound by means of a thiol-ene reaction, also known as alkene hydrothiolation.

With respect to the thiol comprising group, by means of the term "thiol comprising group", reference is made to at least a part of a molecule comprising at least a thiol functional group, in other words an -SH functional group.

It should be clear to the skilled in the art that it derives that the thiol comprising group can either be attached to the compound or the end-capping group, and that the -ene group of formula (I) can either be attached to the compound or the end-capping group. It should also be clear to the skilled in the art that when the thiol comprising group is attached to the compound, the -ene group of formula (I) is attached to the end-capping group.

In accordance with a preferred embodiment of the present invention in the combination the polyester comprises the urethane moiety and the first crosslinkable group is the -ene group moiety of formula (I). In other words, the first crosslinkable group, which is connected to the end-capping group, and therefore part of the polyester, is the -ene group moiety of formula (I), wherein the end-capping group is a urethane moiety. It derives that the second crosslinkable group, comprised in the compound, is the thiol comprising group.

In accordance with a further embodiment, wherein the second crosslinkable group is the thiol comprising group, in the combination the compound comprising the second crosslinkable group is selected from: 1,2-Ethanedithiol, 1,3-Propanedithiol, 1,4-Butanedithiol, 2,2'-Thiodiethanethiol, 2,2'-(Ethylenedioxy)diethanethiol, Trimethylolpropane tris(3-mercaptopropionate), Pentaerythritol tetrakis(3-mercaptopropionate), 2-hydroxymethyl-2-methyl-1,3-propanediol tris-(3-mercaptopropionate) and combinations thereof.

In accordance with the present invention, the compound comprising the second crosslinkable group can be a macromolecule, such as a polymer or copolymer, such as a polyether (e.g PEG) or polyester (e.g. PLA, PLGA, PCL). In accordance with an embodiment of the present invention, the compound comprising the second crosslinkable group is a polyester. In accordance with a further embodiment, the compound comprising the second crosslinkable group is a polyester as described in accordance with the present invention.

In accordance with the present invention, by means of the term "-ene group", reference is made to at least a part of a molecule comprising a carbon double bond, in other words, a C-C double bond, in other words a C=C bond.

Further, in accordance with the present invention, the -ene group of formula (I) is in accordance with:

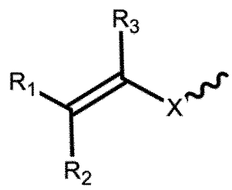

(I)

Wherein $R_1$, $R_2$, $R_3$ are independently selected from: H, alkyl, O, N, halogen, S or at least one of $R_1$, $R_2$, $R_3$ together with the group at position X form a cyclic or heterocyclic structure, and X is a group selected from: alkyl, O, N, S, (C=O)N, (C=O)alkyl, O-alkyl, N-alkyl, S-alkyl. In formula (I), X is a group that is therefore not a carboxylic group, -(C=O)-O-. For example, the -ene group is not an acrylate.

In accordance with a further embodiment of the present invention in the combination the -ene group of formula (I) is selected from: alkene, norbornene, allyl ether, vinyl ether, maleimide, preferably alkene, norbornene, allyl ether, vinyl ether. Therefore, the -ene group can for example a terminal group, part of a cyclic or heterocyclic structure.

Further, in accordance with the present invention the polyester and the compound comprise at least 2 of a first, respectively second crosslinkable groups, and the sum of said first, respectively second crosslinkable groups, is at least 5. More specifically, in order for the combination to be suitable to crosslink, the polyester and the compound are adapted to provide a crosslinked network, and therefore shall be provided to react together and crosslink, and to do so, the first and second crosslinkable groups shall be selected based on their capability of reacting with each other and propagate a network. In order to provide for a crosslinked network, the polyester and the compound have to comprise at least 2 of a first, respectively second crosslinkable groups, and the sum of said first, respectively second crosslinkable groups, is at least 5. Suitable examples of crosslinkble compositions in accordance with the present invention are compositions wherein the polyester comprises at least 2 thiol comprising groups, and the compound comprises at least 3 -ene groups, or vice-versa,

meaning that the polyester comprises at least 3 -ene groups and the compound comprises at least 2 thiol comprising groups.

In accordance with a further embodiment of the present invention, in the combination, the molar ratio, therefore the ratio of number of first crosslinkable groups to number of second crosslinkable groups, e.g. of the -ene group of formula (I) to the thiol comprising group, is from 0.75 to 1.5, preferably from 0.8 to 1.2..

Combinations in accordance with the present invention and the crosslinked networks therefrom obtained provide for fine-tunable bioresorbable networks especially useful in regenerative tissue engineering, due to their improved mechanical characteristics compared to the state of the art. More specifically, it has been found that crosslinked polyesters in accordance with the present invention have elongation at break and ultimate strength higher than the one of acrylate-based crosslinked polyesters. These beneficial properties are believed to be the result of a synergistic effect given by the crystallinity of the polyester and the homogeneous network topology obtained.

In accordance with a further embodiment of the present invention, the molar polyester to compound ratio in the composition is selected based on the molar ratio of the first crosslinkable group and second crosslinkable group, so that the ratio of the total number of first crosslinkable groups to total number of second crosslinkable groups in the composition is equal to 1. For example, in accordance with the present embodiment, if the polyester comprises 2 first crosslinkable groups being -ene comprising groups, and the compound comprises 4 second crosslinkable groups being thiol comprising groups, the molar polyester to compound ratio is 2:1, selected based on the respective ratio of the total number of first crosslinkable groups (2 moles of polyester required to have 4 total first crosslinkable groups) to total number of second crosslinkable groups (just one mole of compound required) in the composition being equal to 1 (4:4) (2x moles comprising 2 first crosslinkable groups):(1 mole of compound comprising 4 thiol comprising groups).

For example, in accordance with the present invention, polycaprolactone (PCL) is functionalized via chemical modification, with alkene (-ene) photo-reactive end groups, and a synergistic effect of the crystallinity of PCL and the homogeneous network topology, acquired via thiol-ene crosslinking, is observed. As a result, thiol-ene photo-crosslinked PCL with an elongation at break of 736.3 % $\pm$ 47 % and ultimate strength of 21.3 MPa $\pm$ 0.8 MPa is obtained, which is significantly higher compared to acrylate-based photo-crosslinkable PCL currently described in the state-of-the-art. Therefore, the herein reported materials have the potential to greatly influence the application of laser-based additive manufacturing for biomedical applications.

In accordance with a preferred embodiment of the present invention in the combination the polyester comprises at least 2 end-capping groups comprising each at least one -ene group moiety of formula (I). For example, if the polyester is a linear polyester, therefore comprising a linear backbone, 2 end-capping groups, such as urethane, can be provided to be attached to each end of the linear polyester, wherein each end capping group, at each end, comprises at least one -ene group moiety of formula (I), such as a terminal alkene, such as vinyl ether.

In accordance with an embodiment of the present invention in the combination the polyester is of formula (B)

$$((A_m)_y - Y_m - Z_m)_n \text{ - backbone (B)}$$

wherein:

- $((A_m)_y - Y_m - Z_m)_n$ represents at least one end-capping group, and
- $A_m$ represents the first crosslinkable group;
- $Y_m$ is selected from the list comprising: the direct bond or the spacer;
- $Z_m$ represents the moiety selected from ester, urethane, thiocarbamate or urea, connected to said backbone, directly or indirectly (e.g. by means of a spacer); and

wherein n is integer and defines the number of arms/branches connected to the backbone, with $n \geq 1$, and wherein y is an integer and defines the number of first crosslinkable groups, and m is an enumerator for each arm/branch. For example, the present invention can be put into practice by providing a combination comprising a modified PCL and a pentaerythritol tetrakis(3-mercaptopropionate) (compound comprising the second crosslinkable group), wherein the modified PCL according to the present invention is represented at the bottom of **Fig. 1** (EUP PCL). EUP PCL comprises a polycaprolactone backbone, to which two end-capping groups (n=2) are attached. Each end-capping group comprises a urethane moiety ($Z_m$) connected to a first crosslinkable group ($A_m$), which is a single -ene group (y=1), more specifically a $CH_2$=$CH_2$-, connected to the urethane moiety by means of a spacer ($Y_m$), in this case a -$CH_2$- group. The urethane moiety is further indirectly connected to the PCL backbone by means of an isophorone group and a urethane moiety.

In a second aspect, the present invention relates to a crosslinked polyester comprising a combination as defined in accordance with the present invention wherein the polyester and the compound form a crosslinked network.

In a third aspect, the present invention relates to a process to produce a crosslinked polyester according to the present invention, comprising the steps of:

a) providing a combination as defined in accordance with the present invention,
b) crosslinking the combination provided at step a).

In accordance with an embodiment of the present invention, in the process step a) further comprises providing an initiator to the combination, such as a photoinitiator, a thermal initiator or a redox initiator. Alternatively, the crosslinking of step b) can also be accomplished by means of plasma-induced polymerization.

In a third aspect, the present invention relates to a use of a combination as defined in accordance with the present invention and/or a crosslinked polyester as defined in accordance with the present invention, in additive manufacturing as a bioink, bioengineering, biomedical applications, such as implants, preferably biodegradable implants, such as bone implants, cartilage implants.

**EXAMPLE 1 - EUP PCL + PETA 4SH**

The present example pertains to a combination and crosslinked polyester (EUP PCL + PETA 4SH) in accordance with the present invention, wherein the combination comprises a modified poly-ε-caprolactone (PCL) and a pentaerythritol tetrakis(3-mercaptopropionate) (PETA 4SH), wherein the modified PCL is an ene-terminated urethane-based polyester (EUP), see **Fig. 1.**

In accordance with the present example, the end-capping group comprises a first crosslinkable group being an -ene group of formula (I), wherein $R_1$, $R_2$, $R_3$ are H, and X is an alkyl ($CH_2$) connected to a urethane moiety (-O(C=O)NH-).

**Results and discussion**

**Synthesis of the functionalized photo-reactive PCL precursors**

In order to render PCL photo-crosslinkable, end group functionalization with different photo-reactive moieties, namely, acrylates, alkenes and alkynes, has been performed via urethane coupling chemistry, see **Fig. 1. Fig. 1** illustrates the two-step synthesis protocol of the acrylate-(AUP PCL), alkene- (EUP PCL) and alkyne- (YUP PCL) terminated PCLs. In the first step, the endcaps with acrylate, alkene and alkyne functionalities were synthesized via the nucleophilic addition reaction of isophorone diisocyanate (IPDI) and 2-hydroxy ethyl, allyl alcohol and propargyl alcohol, respectively. In the second step, PCL diol with a molar mass of 2000 $g.mol^{-1}$, was reacted with the respective endcaps in order to obtain acrylate, alkene and alkyne terminated PCL. These compounds will be further referred to as acrylate-terminated urethane-based polymer (AUP PCL), ene-terminated urethane-based polymer (EUP PCL) and yne-terminated urethane-based polymer (YUP PCL), respectively.

Via quantification of the isocyanate functionalities through the back titration using dibutyl amine, the reaction progress was monitored. Furthermore, completion of the reaction could be confirmed by FTIR as the characteristic isocyanate stretching vibration at 2260 cm-1 completely disappeared. Successful functionalization with degrees of substitution of 73, 63 and 80 was confirmed for the synthesized acrylate (AUP PCL), alkene (EUP PCL) and alkyne (YUP PCL) functionalized PCL, respectively, through quantitative 1H-NMR with dimethyl terephthalate (DMT) as internal standard.

**Design of different network topologies**

Based on the synthesized oligomeric precursors, four networks with different network topologies were prepared as a result of the chemistries involved, see **Fig. 2. Fig. 2** illustrates a schematic representation of the different topologies of the PCL-based networks, stemmed from the involved crosslinking chemistries; A. Acrylate crosslinking (AUP PCL); B. Acrylate-thiol binary crosslinking (AUP PCL + PETA 4SH); C. Thiol-ene crosslinking (EUP PCL + PETA 4SH); D. Thiol-yne crosslinking (YUP PCL + PETA 4SH). In order to photo-crosslink the respective networks, 2m% ethyl (2,4,6-trimethylbenzoyl) phenylphosphinate (TPO-L, according to the amount of terminal functionalities) has been incorporated prior to UV-A irradiation (30 mins, 10 $mW/cm^2$). A first network was prepared via crosslinking of AUP PCL, without an additional crosslinker, resulting in a purely chain growth polymerization of the acrylate functionalities, see **Fig. 2A.** In order to shift the chain growth polymerization to a binary system in which both step and chain growth polymerization are combined, AUP PCL has been combined with (pentaerythritol tetrakis(3-mercaptopropionate)) (PETA 4SH). PETA 4SH is a tetrafunctional thiol which can act as a chain transfer agents, thereby shifting the polymerization from purely chain growth to a combination of both step and chain growth polymerization, see **Fig. 2B.** If the polymerization will occur via a chain or step growth mechanism depends on whether the ene moiety is able to undergo radical homopolymerization. Enes such as allyl ethers, vinyl ethers and norbornenes do not homopolymerize, while acrylate and methacrylates do. Furthermore, the extent of step or chain growth depends on the ratio of the kinetic rate constants for the propagation and chain transfer reactions. Notwithstanding that the ratio of the thiol propagation to the homopolymerization rate is close to 8, an acrylate to thiol ratio

of 2:1 was selected in order to prevent loss of the network integrity. Furthermore, a complete step growth polymerized network based was designed via the combination of EUP PCL and PETA 4SH, see **Fig. 2C,** which is in accordance with the present invention. Based on the orthogonality of the thiol-ene reaction, an alkene to thiol ratio of 1:1 was selected, this being the ratio of the total number of first crosslinkable groups (-ene group of formula (I), in this case vinyl) to total number of second crosslinkable groups (thiol comprising group, in this case the thiol) in the composition. Finally, a fourth network was prepared via the thiol-yne reaction upon the combination of YUP PCL and PETA 4SH, see **Fig. 2D.** Similar to the thiol-ene network, the polymerization occurs via a step growth mechanism network, however, since an alkyne functional group subsequently reacts with two thiol functionalities, the crosslinking density will be twice that of the thiol-ene based network (EUP PCL+ PETA 4SH). Therefore, an alkyne to thiol ratio of 1:2 was selected for the preparation of the thiol-yne network (YUP PCL + PETA 4SH).

**Network integrity and photo-reactivity**

In order to investigate the network integrity, gel fraction and swelling experiments have been performed, see **Table 1.** Gel fractions above 80 % have been retrieved for all networks indicating good network integrity and successful network formation. The differences in gel fractions can be attributed to the different degree of substitutions obtained during the synthesis. As swelling experiments hold information about the network topology and crosslink density they can give a valuable first impression of the influence of the different crosslinking chemistries. The lowest swelling ratio of 6.2 $\pm$ 0.1 was retrieved for the thiol-yne crosslinked network (YUP PCL + PETA 4SH). This could be expected based on the fact that one alkyne functionality will react with two thiol functionalities, thereby leading to an increased crosslink density. It can be assumed that this increased crosslink density leads to the formation of a more rigid network and therefore reduces the swelling capacity. Furthermore, swelling ratios for the acrylate containing networks of 7.2 $\pm$ 0.1 (AUP PCL) and 12.7 $\pm$ 0.2 (AUP PCL + PETA 4SH) were found without and with the addition of the tetrafunctional thiol (PETA 4SH), respectively. These results reveal that incorporation of the tetrafunctional thiol (PETA 4SH), leads to a significantly increased swelling capacity of the networks. This effect can be attributed to the more homogeneous network topology that is obtained via the combination of chain and step growth polymerization as a result of the introduced thiol. Furthermore, a swelling ratio of 10.6 $\pm$ 0.1 was retrieved for the thiol-ene based network (EUP PCL + PETA 4SH). Also here, an increase in the swelling ratio, compared to the AUP PCL network, was found. This effect can also be attributed to the more homogeneous network topology that is obtained via the thiol-ene crosslinking. Finally, in order to determine whether quantitative conversion of the photo-reactive groups has taken place, the networks were evaluated through HR-MAS. HR-MAS allows to assess crosslinked samples and can therefore be used to investigate the fraction of residual acrylates post-crosslinking. Quantitative conversion of the photo-reactive moieties is of utmost importance since remaining unreacted functional groups, such as acrylates, can be cytotoxic and can cause foreign body responses leading to inflammation. Evaluation of the characteristic peaks corresponding to the acrylate, alkene and alkyne functionalities indicated complete conversion.

**Table 1:** Gel fraction, swelling ratio and substitution degree obtained for the different PCL-based networks.

| | Gel fraction (%) [a] | Swelling ratio [a] | Substitution degree (%) |
|---|---|---|---|
| AUP PCL | 92.2 $\pm$ 0.7 | 7.2 $\pm$ 0.1 | 73 |
| AUP PCL + PETA 4SH | 88.4 $\pm$ 1.3 | 12.7 $\pm$ 0.2 | 73 |
| EUP PCL + PETA 4SH | 80.9 $\pm$ 2.2 | 10.6 $\pm$ 0.1 | 63 |
| YUP PCL + PETA 4SH | 86.8 $\pm$ 0.6 | 6.2 $\pm$ 0.1 | 80 |
| a) all experiments have been performed in $CHCl_3$ | | | |

The photo-reactivity of the oligomeric precursors is of utmost importance since it will influence the polymerization kinetics and can affect the ability to process the materials via laser-based 3D-printing techniques such as two-photon-polymerization (2PP), stereolithography (SLA) and digital light processing (DLP). For example, oligomers with high-photo-reactivity will need reduced irradiation times and lower laser intensities to photo-crosslink. This will consequently reduce the manufacturing time and improve the cost-efficiency of the technique.

Via photo-rheology, the influence of the different crosslinking chemistries on the network formation of the oligomeric precursors has been investigated, see **Fig. 3. Fig. 3** illustrates the photo-rheology of the different PCL-based networks. Two distinct effects can be seen. First, the thiol-ene and thiol-acrylate crosslinking revealed to be faster compared to the thiol-yne crosslinking. Furthermore, the final loss modulus (G’), obtained after photo-crosslinking, seems to depend on the involved crosslinking chemistry. The crosslinked polyesters in accordance with the present invention, obtained from the combination EUP PCL + PETA 4SH provided the highest post-polymerization storage modulus, showing an improvement over acrylate comprising crosslinked polyesters (AUP PCL + PETA 4SH). Furthermore,

incorporations of thiols lowered the post-polymerization storage modulus. Finally, the lowest post polymerization modulus was found for the thiol-yne crosslinking. However, this can be attributed to the slow photo-reactivity since the storage modulus kept increasing over the next 2000 seconds finally reaching a similar value as the thiol-acrylate network.

## Thermal properties

Thermogravimetric analysis (TGA) and differential scanning calorimetry (DSC) were employed to investigate the influence of the network topology on the thermal properties, see **Table 2.**

**Table 2:** Thermal properties of the different networks.

| | $T_g$ (°C) b | $T_d$ (°C) | $T_c$ (°C) b | $\Delta H_c$ (J/g) b | $T_m$ (°C) b | $\Delta H_m$ (J/g) b | X (%) |
|---|---|---|---|---|---|---|---|
| AUP PCL | -59.9±0.5 | 238.7 | -17.9±1.2 | 3.6±0.6 | 35.4±0.2 | 35.7±1.1 | 26 |
| AUP PCL + PETA 4SH | -55.5±0.3 | 256.8 | -15.4±0.4 | 4.9 ± 0.4 | 38.5±0.1 | 40.8±0.6 | 30 |
| EUP PCL + PETA 4SH | -53.8±0.2 | 258.2 | -15.7±0.3 | 9.1 ± 1.0 | 40.0±0 | 37.8±0.7 | 28 |
| YUP PCL + PETA 4SH | -52.6±1.8 | 219.5 | ND a | ND a | 39.4±0.2 | 1.4±0.2 | 1 |
| a) ND = not detected. b) reported values are the average of triplicates ± standard deviation. | | | | | | | |

Degradation temperatures (determined at the onset) of 366, 366, 358 and 355 °C were determined for the acrylate (AUP), acrylate-thiol (AUP + PETA 4SH), thiol-ene networks in accordance with the present invention (EUP + PETA 4SH) and thiol-yne (YUP + PETA 4S) based networks, respectively. These degradation temperatures are in line with reported values in the literature for PCL and do not seem to be altered by the involved crosslinking chemistry. In the context of their biomedical application, it can be stated that all the networks have appropriate temperature stabilities. Additionally, glass transition temperatures of - 60, - 56, -54 and - 53 °C were retrieved for the acrylate (AUP PCL), acrylate-thiol (AUP + PETA 4SH), thiol-ene (EUP + PETA 4SH) and thiol-yne (YUP + PETA 4SH) based networks, respectively. Once more, these values are in good agreement with reported values for linear PCL, and thus, rubbery networks are obtained at room temperature. Interestingly, although appropriate network formation has been confirmed, after photo-crosslinking, all the PCL-based networks retain sufficient chain mobility to allow for melting and recrystallization, see **Fig. 4. Fig. 4** illustrates DSC thermograms of the PCL-based networks (first from the top - AUP PCL; second from the top - AUP PCL + PETA 4SH; third from the top - EUP PCL + PETA 4SH; bottom - YUP PCL + PETA 4SH).

Upon further investigation of the melting and crystallization behavior for the various networks, peculiar behavior has been observed. Although the melting temperature (35.7 °C) determined as the maximum of the transition is considerably higher than room temperature, the melting behavior appeared to be altered if the samples were stored at room temperature. When stored below room temperature (4 °C), a single melting peak with similar melting enthalpies for all materials were retrieved as can also be observed in the thermograms depicted in **Fig. 5. Fig. 5** illustrates DSC thermograms showing the initial crystallinity (after storage at room temperature for one week) of the four PCL-based networks (first from the top - AUP PCL; second from the top- AUP PCL + PETA 4SH; third form the top- EUP PCL + PETA 4SH; bottom - YUP PCL + PETA 4SH). This can be expected since the thermal history of the materials has been erased during the second heating run. However, if the materials were stored at room temperature, the melting enthalpy changed and two distinct melting peaks were retrieved, instead of one, see **Fig. 5**. It is assumed that this behavior can be attributed to melt recrystallization phenomena, able to occur at room temperature. As a result, a fraction of the crystallized PCL can be molten and recrystallized, thereby forming the observed second melting peak. The initial crystallinity (that is after storing the materials at room temperature) was significantly higher in case of the more homogeneous network topology. Based on this observation, it is assumed that the more homogeneous network topology of the thiol-ene based network, in accordance with the present invention, (EUP PCL + PETA 4SH) allows recrystallization to a higher extend, thereby not resulting in a reduction of the melting enthalpies. In contrast, the acrylate based network (AUP PCL), shows a significant reduction in initial melting enthalpy of ~13 $J.g^{-1}$ after being stored at room temperature. It is assumed that its inhomogeneous network topology does not allow recrystallization to the same degree as the more homogeneous network topologies, resulting in net melting enthalpy reduction. As the acrylate-thiol based network (AUP PCL + PETA 4SH) is formed via a combination of both chain and step growth polymerization, it can be expected that the observed behavior is a combination of both the previous behaviors. As can be seen in **Fig. 5,** this is in line with the expectations revealing a melting enthalpy reduction of ~ 9 $J.g^{-1}$. Although for the thiol-yne based network (YUP PCL + PETA 4SH) crystallization was not observed within the timeframe of the performed DSC experiments, it could be observed that within a longer timeframe this network crystallized as well. Furthermore, a lower reduction in the melting enthalpy after storage at room temperature was retrieved (~ 6 $J.g^{-1}$). However, due to the highly rigid network topology resulting in the increased crosslink density, recrystallization could not at all be observed. During the second heating run, the crystallinity of all networks ranged

between 26 and 30 %, except for the alkyne-based PCL which is due to the slow crystallization compared to the timeframe of the performed DSC experiments. However, crystallinities of 16, 22, 27 and 25 % are obtained when the calculation is based on the initial crystallinity (after storage at room temperature).

**Mechanical properties**

Finely-tuned mechanical properties are of utmost importance in tissue engineering in order to provide sufficient mechanical support and to prevent stress-shielding. For example, if the scaffold is stronger than the native tissue, it will prevent the surrounding tissue to be mechanically loaded. This can influence the healthy homeostatic balance of the cells and lead to deterioration of the tissue which is a process termed stress-shielding. Furthermore, it is known that the modulus of the material can influence the behavior of cells including proliferation and differentiation towards certain cell-lines.

Via tensile testing of the dog bone shaped test specimens (20 mm gauge length, 0.5 mm gauge thickness, 4 mm gauge width) the Young's modulus, ultimate strength and elongation at break of the different networks were determined, see **Table 3.** These results revealed that the different network topologies have an effect on the mechanical properties of the materials. It can be seen that the Young's modulus increases for the more homogeneous network topologies. Since non-crystallized samples exhibited a Young's modulus of only 4 MPa, it is assumed that the Young's modulus is mainly governed by the crystallinity of the materials. This assumption can be further stated by the good agreement between the trend in the Young's moduli and the initial melting enthalpy and crystallinity obtained after storage at room temperature, see **Fig. 5**. In the context of tissue engineering, this can be an interesting feature since it could be used as tool to fine-tune the mechanical properties towards a specific tissue. However, this effect is herein not further investigated. Looking at the elongation at break and the ultimate strength, a similar trend can be observed, see **Fig. 6. Fig. 6** illustrates stress-strain plots obtained via tensile testing for the different crosslinked networks: AUP, AUP PCL + PETA 4SH, EUP PCL + PETA 4SH and YUP PCL + PETA 4SH. More specifically, the more homogeneous the network topology, the higher the elongation at break, and consequently, the ultimate strength. It should be noted that this effect is very well reflected in the case of the thiol-ene based network in accordance with the present invention (EUP PCL + PETA 4SH), which theoretically is a perfectly homogeneous network. As a result, an ultimate strength of 21.3 ± MPa and an elongation at break of 736.3 ± 47 was found, which are a multifold greater compared to acrylate-terminated PCLs, currently the reported state-of-the-art. It should be noted that the obtained properties can be compared to linear PCL with a molar mass of 80 000 $g.mol^{-1}$ which is widely reported in extrusion-based 3D-printing for biomedical applications. Consequently, this material has the potential to greatly influence the application of laser-based additive manufacturing for biomedical applications. It is assumed that the improved mechanical properties are the result of a synergetic effect of the crystallinity of the material and the homogeneous network topology obtained via the thiol-ene crosslinking. Finally, it can be seen that due to the high crosslink density in case of the thiol-yne based network (YUP PCL + PETA 4SH), the elongation at break (36 ± 17%) and ultimate strength (4.1 ± MPa) are significantly lower compared to the thiol-ene based network of the present invention, as a result of the higher crosslink density. Therefore, although counterintuitive, a higher crosslink density does not necessarily mean better mechanical properties. Especially in a biomedical context, where materials should be easy to handle by the surgeon, brittle networks obtained via an increase in the crosslink density might not be beneficial.

**Table 3:** Young's moduli, elongation at break, ultimate strength and toughness obtained via tensile testing of the photo-crosslinked networks.

| | Young's modulus (MPa) | Elongation at break (%) | Ultimate strength (MPa) | Toughness ($MJ.m^{-3}$) |
|---|---|---|---|---|
| AUP PCL | 38.7 ± 4.1 | 177 ± 45 | 7.5 ± 1.6 | 886 ± 345 |
| AUP PCL + PETA 4SH | 50.6 ± 2.3 | 514 ± 104 | 13.6 ± 2.2 | 4329 ± 1416 |
| EUP PCL + PETA 4SH | 93.4 ± 5.3 | 736 ± 47 | 21.3 ± 0.8 | 8660 ± 245 |
| YUP PCL + PETA 4SH | 70.5 ± 4.0 | 36 ± 17 | 4.1 ± 0.3 | 130 ± 76 |
| a) reported values are the average of triplicates ± standard deviation. | | | | |

**In vitro biocompatibility**

In the context of tissue engineering, biocompatibility is of utmost importance as this is a crucial aspect in order to bring a biomedical implant onto the market. Therefore, in order to investigate whether the materials are biocompatible, neutral red uptake experiments were performed, see **Fig. 7. Fig. 7** illustrates neutral a red uptake experiment according to ISO standard 10993 in order to investigate the biocompatibility of the four PCL-based networks. According to the ISO standard 10993, if cell viabilities exceed 70 %, obtained via this test, the respective materials can be considered

biocompatible. As depicted in **Fig. 7**, the cell viabilities exceed 70 % for all the four different PCL-based network indicating that the materials are in fact biocompatible. The highest cell viabilities were retrieved for the acrylate (AUP PCL) and thiol-yne (YUP PCL + PETA 4SH) based networks. For these networks the highest gel fractions have been determined, indicating a possible cause for the slightly lower cell viabilities obtained in the case of the acrylate-thiol (AUP PCL + PETA 4SH) and thiol-ene (EUP PCL + PETA 4SH) based networks.

**Conclusion**

In summary, the network topology has been introduced as a strategy to optimize the properties of photo-crosslinkable bioresorbable PCL networks. Acrylate, alkene and alkyne-terminated PCL were synthesized, characterized and their in vitro biocompatibility was evaluated. Controlling the network topology proved to be an efficient method to greatly enhances the elasticity and toughness of resorbable PCL networks. As a result of the superior mechanical properties, the herein reported materials have the potential to greatly influence the application of laser-based additive manufacturing for biomedical applications.

**Experimental Section**

*Materials:*

Poly-ε-caprolactone diol ($M_n$ = 2000 g.mol$^{-1}$, Sigma Aldrich), chloroform (> 99.5%, Chem-LAB), isophoronediisocyanate (IPDI, 98 %, Sigma Aldrich), ethyl (2,4,6-trimethylbenzoyl) phenylphosphinate (TPO-L speedcure), 1-methyl-2-pyrrolidone (NMP, 99%, Sigma Aldrich), dimethyl terephthalate (DMT, TraceCERT, Sigma Aldrich), HCL (1N in isopropanol, Fischer Sci), pentaerythritol tetrakis(3-mercaptopropionate) (PETA 4SH, > 95 %, Sigma Aldrich), 2-hydroxyethyl acrylate (96 %, Sigma Aldrich), dibutylamine (99.5 %, Sigma Aldrich), allyl alcohol (> 99 %, Sigma Aldrich), propargyl alcohol (> 99 %, Sigma Aldrich) were used as received. Bismuth neodecanoate and phenothiazine (PTZ) were provided by Allnex (Belgium). Toluene (> 99.9 %, Chem-lab) was refluxed over sodium with benzophenone as indicator and distilled before use.

*Synthesis of the functionalized PCLs:*

IPDI (1eq., 30.1335 g, 0.136 mol) was weighed in a flame-dried two-neck flask under argon atmosphere. PTZ (15 mg, mmol) and bismuth-neodecanoate (15 mg, mmol) were added as inhibitor and catalyst respectively. Subsequently, the mixture was heated to 60 °C followed by dropwise addition of 2-hydroxyethyl acrylate (1 eq., g, mmol), allyl alcohol (1 eq., g, mmol) or propargyl alcohol (1 eq., g, mmol) for the synthesis of AUP PCL, EUP PCL and YUP PCL, respectively. After addition, the mixture was left to stir for 1 hour. Isocyanate quantification (vide infra) using dibutylamine indicated that the reaction was finished. The IPDI adduct was subsequently cooled in an ice-bath and directly used in the following step without further purification. In the second step, PCL diol (0.5 eq., 2000 g.mol-1) was dissolved in toluene in a flame-dried two-neck flask equipped with a stirring bar. To the solution were added PTZ (15 mg, mmol) and Bismuth neodecanoate (15 mg, mmol) after which the mixture was heated to 60 °C. Subsequently, the IPDI adduct prepared in the previous step was added dropwise to the solution. After addition, the reaction temperature was increased to 80 °C and the mixture was left to react overnight. Via FTIR, complete conversion of the isocyanates could be confirmed since the characteristic stretching vibration of the -NCO's was completely disappeared. Finally, AUP PCL, EUP PCL and YUP PCL were purified via precipitation in cold diethyl ether with a yield of 64 %, 63 % and 70 %, respectively.

*Determination of the degree of substitution:*

The degree of substitution was determined via quantification of the acrylate, alkene and alkyne functionalities, here referred to as the functional content (mmol.g$^{-1}$). In order to do so, $^1$H-NMR samples were prepared in $CDCl_3$ containing 10 mg of the respective PCL-derivatives and dimethyl terephthalate (10 mg, 0.051 mmol) as internal standard. Subsequently, the double bond content was calculated using the following formula:

$$Functional\ content\ (mmol.g^{-1}) = \frac{(characteristic\ peaks\ )}{I_8} \times \frac{n_{double}}{n_{DMT}} \times \frac{m_{DMT}}{M_{DMT}} \times \frac{1}{m_{PCL}} \qquad (1)$$

In this equation, $n_{double}$ and $n_{DMT}$ refer to the number of protons of the integrated signals corresponding to the acrylate/alkene/alkynes and DMT (I = 8), $m_{DMT}$ and $M_{DMT}$ refer to the weighed mass and the molecular weight of DMT, $m_{PCL}$ refers to the weighed mass of the modified PCL. $I_8$ refers to the intensity for the peak at 8 which corresponds to the aromatic protons of DMT (n = 4). Finally the characteristic peaks employed for the different modified PCLs are the

following: AUP PCL ($I_{6.40}$, $I_{6.12}$ and $I_{5.83}$), EUP PCL ($I_{5.9}$, $I_{5.2}$ and $I_{5;3}$) and YUP PCL: ($I_{2.8}$). The obtained double bond molar concentration was then used to calculate the degrees of substitution according to the theoretical molar mass of the final products (AUP PCL = 73%, EUP PCL = 63% and YUP PCL = 90%).

*Isocyanate quantification:*

The isocyanate content was determined via back-titration with dibutylamine using an automatic titrator equipped with 1 M HCl. First, a solution containing 64.5 g dibutylamine (64.5 g, 0.499 mol) in 1L NMP was prepared. Approximately 200 mg of the reaction mixture was reacted with 3 mL of the dibutylamine solution in 50 mL NMP during 15 minutes of stirring. Subsequently, the residual dibutylamine was quantified through automatic titration using HCL (1M in isopropanol). The isocyanate concentration could be calculated using following formula:

$$Isocyanate\ content\ (mmol.g^{-1}) = \frac{(V_{blanc} - V_{sample}) \times C_{HCl}}{m_{sample}} \quad (2)$$

In this equation $V_{blanc}$ and $V_{sample}$ refer to the employed volume of the blanc and the sample respectively, $C_{HCL}$ refers to the concentration of the HCL solution (1M in isopropranol) and $m_{sample}$ refers to the mass of the measured sample.

*Photo-curing*:

AUP PCL, EUP PCL and YUP PCL (4 g, x mol) and TPO-L (2m% according to double bonds) as photo-initiator were dissolved in $CHCl_3$. The solution was vigorously stirred followed by evaporation of $CHCl_3$. Subsequently, the melt was crosslinked under UVA light (315-400 nm, 30 minutes, 10 $mW/cm^2$) in a glass mold equipped with a silicone spacer after which films with a thickness of 0.5 mm were obtained.

*Characterization:*

Structure elucidation was performed through $^1$H-NMR spectroscopy using a Bruker spectrometer (400 MHz) with $CDCl_3$ as solvent and Fourier-transform infrared spectroscopy (FTIR) using a Perkin-Elmer spectrometer. The thermal properties were studied by thermogravimetric analysis (TGA - TA instruments Q50) and differential scanning calorimetry (DSC - TA instruments Q2000). TGA was performed starting from 30 °C up to 600 °C with a heating rate of 10 °C per minute under nitrogen atmosphere. DSC was performed using ~ 5 mg of sample. In the first heating cycle, the sample was heated from 30 °C up to 100 °C and subsequently cooled to - 80 °C. Thereafter, the sample was heated again heated to 100 °C, during the second heating cycle. Heating and cooling rates of 10 °C per minute were employed while being exposed to nitrogen atmosphere. Swelling and gel fraction experiments were performed using 3 mm diameter discs which were punched out from crosslinked films with a thickness of 0.5 mm. The samples were immersed in $CHCl_3$ during 24 hours and subsequently dried under vacuum during 24 hours at 80 °C. Degrees of swelling and gel fractions were calculated using the following formulas:

$$Gel\ fraction = \frac{m_d}{m_i} \times 100\ (\%) \quad (3)$$

$$Degree\ of\ swelling = \frac{m_s - m_d}{m_i} \quad (4)$$

Here $m_d$ refers to the dry mass, $m_s$ to the swollen mass and mi to the initial mass before swelling. Tensile experiments were performed using x, equipped with a load cell of 250 N and Horizon software. An initial strain rate of 0.1 mm per minute was used up to 0.1 % elongation after which the strain rate was increased to 1 mm per minute. Photo-rheology was performed using an Anton Paar rheometer equipped with a UV light-source (365 nm, 3500 $mW/cm^2$).

*In vitro biological evaluation:*

3T3 (Merk, Germany) cells were maintained at 37°C in 5% $CO_2$ in Dulbecco's Modified Eagle Medium (DMEM, Sigma-Aldrich, UK) supplemented with 10% (v/v) foetal calf serum (InvitrogenTM, UK) and 1% (v/v) penicillin/streptomycin (InvitrogenTM, UK). The culture media was changed every third day. Once cells had reached between 90 and 95% confluency, they were mechanically scraped and sub-cultured under the same conditions.

For the NRU assay, 3T3 cells which were seeded into 96-well plates at a density of $1 \times 10^4$ cells/well to form a sub

confluent monolayer. After 48 h, the culture medium was removed, and three different dilutions (100% extract, 50% extract, and 25% extract) of the test compounds in medium were added to the cells and incubated for 24h at 37°C in 5% $CO_2$. Treatment medium was used as the untreated vehicle control. After treatment with the extracts, cells were washed once with PBS, and treated with Neutral Red dye for 3h at RT, followed by NR desorb (ETOH/acetic acid) solution added to all wells. The absorption of the resulting coloured solution was measured at 540 nm in a plate reader. Cell viability was calculated as the percentage of vehicle control (cell culture media only) values. For the treatment extracts, all compounds were extracted at a ratio of $3cm^2$ / ml in vehicle medium for 72 h before being added to cell culture.

**EXAMPLE 2 - END-CAPPING GROUP**

Alkene-functionalized PCL containing 4 urethane functionalities (vide supra), 2 urethane functionalities (EU2P PCL 2000) and 2 ester functionalities (E-PCL 2000) between the backbone and the endcap have been synthesized. In order to synthesize EU2P-PCL 2000, PCL diol (2000 $g.mol^{-1}$, 10 g, 5 mmol, 1 eq.) was added in a flame dried flask under argon atmosphere. Anhydrous toluene and a catalytic amount of bismuth neodecanoate were added. The mixture was heated to 40 °C and allyl isocyanate (MM = 83.09 $g.mol^{-1}$, 20 mmol, 4 eq.) was added dropwise. $^1$H-NMR analysis was employed to follow the reaction progress. After the reaction was finished, the product was purified via precipitation in diethyl ether. The final compound was obtained with a yield of 83 %.

Synthesis E-PCL 2000: PCL diol (2000 $g.mol^{-1}$, 10 g, 5 mmol, 1 eq.), was added in a flame dried flask under argon atmosphere. Anhydrous toluene and triethyl amine (MM = 101.19 $g.mol^{-1}$, 12.3 mmol, 2.5 eq.) were added to the mixture. The mixture was cooled to 0 °C and 4-pentenoyl chloride (MM = 118.16 $g.mol^{-1}$, 16.6 mmol, 3.3 eq.) was added dropwise. The reaction was left stirring overnight and allowed to heat to room temperature. The final product was obtained with 75 % yield after precipitation in diethyl ether.

Subsequently, the described alkene-functionalized PCLs have been combined with pentaerythritol tetrakis(3-mercaptopropionate) in order to obtain thiol-ene photo-crosslinked PCL-based networks in the presence of ethyl (2,4,6-trimethylbenzoyl) phenyl phosphinate as photo-initiator. Mechanical characterization of the networks via tensile testing was performed (Table 4). These results further confirm the superior mechanical properties of the crystalline polyester networks crosslinked via thiol-ene chemistry as indicated by the elongation at break (> 392 %) and ultimate strength (> 13.1 MPa) which is significantly higher compared to state-of-the-art.

**Table 4:** Young's moduli, elongation at break and ultimate strength obtained via tensile testing of the PCL-based network containing 4 urethane, 2 urethane or 2 ester functionalities. DS (degree of substitution) of the networks are similar.

| Urethanes | MW | DS (%) | Young's modulus (MPa) | Elongation at break (%) | Ultimate strength (MPa) |
|---|---|---|---|---|---|
| 0 (2 esters) | 2416 | 86 | 129.8 ± 8.0 | 434 ± 57 | 13.1 ± 1.5 |
| 2 | 2416 | 76 | 175.5 ± 38.0 | 392 ± 27 | 14.4 ± 3.2 |
| 4 | 2416 | 75 | 93.4 ± 5.3 | 736 ± 47 | 21.3 ± 0.8 |

**EXAMPLE 3 - THIOL**

Networks based on EU2P-PCL 2000 and different thiol crosslinker have been prepared. More precisely, EU2P-PCL 2000 has been photo-crosslinked via thiol-ene chemistry in combination with a tetra-functional thiol (pentaerythritol tetrakis(3-mercaptopropionate)) and a tri-functional thiol (trimethylolpropane tris(3-mercaptopropionate)) in presence of ethyl (2,4,6-trimethylbenzoyl) phenyl phosphinate as photo-initiator. The resulting polyester-based networks were mechanically characterized via tensile testing (Table 5). These results further confirm the superior mechanical properties of the crystalline polyester networks crosslinked via thiol-ene chemistry as indicated by the elongation at break (> 317 %) and ultimate strength (> 13.3 MPa) which is significantly higher compared to state-of-the-art.

**Table 5:** Young's moduli, elongation at break and ultimate strength obtained via tensile testing of the PCL-based network constituting a tri-functional thiol crosslinker and a tetra-functional thiol crosslinker. DS (degree of substitution) of the networks are similar.

| MW | Crosslinker | DS (%) | Young's modulus (MPa) | Elongation at break (%) | Ultimate strength (MPa) |
|---|---|---|---|---|---|
| 4622 | 4SH | 98 | 168.1 ± 2.4 | 475 ± 51 | 15.7 ± 1.0 |
| 4516 | 3SH | 99.5 | 204.7 ± 3.2 | 317 ± 25 | 13.3 ± 0.3 |

## EXAMPLE 4 - EFFECT OF VARIATION OF MOLAR MASS, POLYESTER ARCHITECTURE AND THIOL FUNCTIONALITY AND COMPARATIVE EXAMPLE

The present example investigates the effect of variation of molar mass, thiol functionality and polyester architecture in networks obtained in accordance with the present invention. In particular, the present example pertains to alkene-terminated PCL with one urethane-functionality at each chain end which were synthesized according to the protocol depicted in **Fig. 8.** The synthesized alkene-functionalized PCL has been cross-linked via thiol-ene cross-linking using a thiol-based crosslinker (pentaerythritol tetrakis(3-mercaptopropionate) or trimethylolpropane tris(3-mercaptopropionate)). The molar mass of the central PCL-segment has been altered (i.e. 2000, 4000, 6000 and 8000 g/mol). Furthermore, the architecture of the central PCL-segment has been altered (i.e. bifunctional, trifunctional and tetrafunctional). Finally, the thiol-based cross-linker has been altered (i.e. trifunctional and tetrafunctional). Further, the present example discloses mechanical properties of the thiol-ene cross-linked PCLs are compared to their acrylate cross-linked counterparts, with identical molar masses.

### Results and discussion

### Effect of variation of molar mass

The influence of the altered network architecture on the mechanical properties is further elucidated via tensile testing of the photo-crosslinked materials. As shown in **Fig. 9,** increasing the molar mass of the central PCL-segment from 4580 to 6260 and 8850 g/mol, resulted in an increase of the ultimate strength from 13 to 19 and 22 MPa, respectively. The elongation at break increased from 303 to 472 and 715%, respectively.

### Effect of variation of the polyester architecture

If the architecture of the central PCL-segments was changed from bifunctional to tetrafunctional, the elongation at break and ultimate strength were significantly reduced (**Fig. 10**). More precisely, the ultimate strength is increased from 12 to 17 and 23 MPa as the architecture is altered from tetra- to tri- and difunctional, respectively. The elongation at break increased upon decreasing the functionality of the PCL backbone from 214 to 325 and 713 %, respectively. In case of the four arm star-shaped polymer, the Young's modulus and yield strength were reduced.

### Effect of variation of thiol functionality

Variation of the thiol-crosslinker from a tetra- towards a tri-functional thiol led to an increase in the elongation at break and ultimate strength, making the material more tough (**Fig. 11**). More precisely, the elongation at break increased from 343 to 495 % and the ultimate strength from 13 to 19 MPa, respectively. These results show that the mechanical properties of the proposed thiol-ene cross-linked PCLs are highly tunable.

### Comparative Example - Thiol-ene cross-linked PCLs (present invention) compared to acrylate cross-linked PCLs (state of the art)

If the mechanical properties of the thiol-ene cross-linked PCLs are compared to their acrylate cross-linked counterparts, we can see that the ultimate strength, elongation at break, yield strength and Young's modulus are significantly higher in case of the thiol-ene cross-linked materials (**Fig. 12**). For a central backbone of 2000 g/mol, the elongation at break and ultimate strength are 343 % and 13.4 MPa in case of the thiol-ene crosslinked material. Conversely, for the acrylate material with a molar mass of 2000 g/mol, the elongation at break and ultimate strength are 26 % and 2 MPa, respectively. For a molar mass of 8000 g/mol and the thiol-ene cross-linking chemistry, the elongation at break and ultimate strength are 715 % and 25 MPa. In case of the acrylate cross-linking and a molar mass of 8000 g/mol, the elongation at break and ultimate strength are 275 % and 16 MPa. This clearly shows the beneficial effect of the more homogeneous network topology that is obtained via thiol-ene cross-linking as compared to acrylate cross-linking and suggests that the proposed materials have excellent commercialization potential.

### Experimental Section

*Materials:*

All chemicals were used as received, unless stated otherwise. ε-caprolactone (>99%), supplied by Tokyo Chemical Industry (TCI), was dried over calcium hydride (CaH2) and retrieved through vacuum distillation at 120°C.

Glycerol (>99%), allyl isocyanate (98%), ethylene glycol (anhydrous, 99.8%), pentaerythritol (99%), Tin(II) 2-ethylhexanoate (92.5-100%), dimethyl terephthalate (99.93%), pentaerythritol tetrakis(3-mercaptopropionate) (>95%), trimethylolpropane tris(3mercaptopropionate) (>95%) and NaOH (>97%) were supplied by Sigma-Aldrich (Diegem, Belgium). Glycerol was dried over calcium hydride ($CaH_2$), retrieved through vacuum distillation at 180°C. Ethyl(2,4,6-trimethylbenzoyl) phenyl phosphinate (Speedcure TPO-L, 94.5%) was supplied by Lambson Ltd HQ (West Yorkshire, UK). Toluene (>99%), chloroform (stabilised with amylene, >99%) and diethylether (stabilised with 5-7 ppm BHT, >99%) were supplied by Chem-lab NV (Zedelgem, Belgium). Toluene was dried over molecular sieves (4Å). Deuterated chloroform (stabilised with silver foils + 0.03% TMS, 99.8%) was supplied by Eurisotop.

*Synthesis of EU(1)P PCL(2) with varying molar mass:*

Poly-ε-caprolactone diol (2000 $g.mol^{-1}$) was synthesized using the following procedure. A Schlenk equipped with a magnetic stirrer was flame-dried prior to the start of the experiment. ε-caprolactone (20 g, 0.175 mol, 1eq, M = 114.14 $g.mol^{-1}$), $Sn(Oct)_2$ catalyst (0.1 g, 0.247 mmol 0.5wt% of ε-caprolactone, 405.122 $g.mol^{-1}$), ethylene glycol initiator (0.639 g, 0.0103 mol, 1:17 stoichiometric ratio initiator to monomer, 62.07$g.mol^{-1}$) and anhydrous toluene (23.75 mL, 4 mol/L, 92.14 $g.mol^{-1}$) were added into the Schlenk while being under argon atmosphere. Three freeze-pump-thaw cycles were performed by freezing the solution in liquid $N_2$. After fully freezing the solution, a vacuum was applied, causing the headspace above the frozen solution to be removed. The reaction proceeded for 24h at 100°C while stirring, under argon atmosphere. The reaction continued until $^1$H-NMR spectroscopy verified that the reacted was completed. Subsequently, the obtained PCL diol was modified to photo-crosslinkable alkene-functionalized PCL (EUP-PCL). The exact molar mass was calculated with $^1$H-NMR spectroscopy and based on this value, an excess of 2 eq. allyl isocyanate (1.55 g, 9.35 mmol, 2 eq., MM = 82.09 $g.mol^{-1}$ ) was added for each hydroxyl group. The solution was stirred for 1h at 60°C. $^1$H-NMR spectroscopy was used in order to control the progress of the transition of the hydroxyl functionalities to an alkene end group. Ultimately, the EUP-PCL product was purified through precipitation in cold diethylether, while fast stirring followed by filtration. The synthesis of telechelic EUP-PCL with a molar mass of 4000, 6000, 8000 and 10 000 $g.mol^{-1}$ was similar, with the exception that the initiator to monomer ratio was adjusted to 1:35, 1:52, 1:70, 1:87, respectively.

*Synthesis of tri-functional and tetra-functional EU(1)P PCL:*

Poly-ε-caprolactone triol (8000 $g.mol^{-1}$) was synthesized using the following procedure. A Schlenk equipped with a magnetic stirrer was flame-dried prior to the start of the experiment. ε-caprolactone (20 g, 0.175 mol, 1eq, M = 114.14 $g.mol^{-1}$), Sn(Oct)2 catalyst (0.1 g, 0.247 mmol 0.5wt% of ε-caprolactone, 405.122 $g.mol^{-1}$), glycerol initiator (0.233 g, 2.53 mmol, 1:69 stoichiometric ratio initiator to monomer, 92.09 $g.mol^{-1}$) and anhydrous toluene (24 mL, 4 mol/L, 92.14 $g.mol^{-1}$) were added into the Schlenk while being under argon atmosphere. Three freeze-pump-thaw cycles were performed by freezing the solution in liquid $N_2$. After fully freezing the solution, vacuum was applied, causing the headspace above the frozen solution to be removed. The reaction proceeded for 24h at 100°C while stirring, under argon atmosphere. The reaction continued until $^1$H-NMR spectroscopy verified that the reaction was finished. Subsequently, the obtained PCL diol was modified to photo-crosslinkable alkene-functionalized PCL (EUP-PCL). The exact molar mass was calculated with $^1$H-NMR spectroscopy and based on this value an excess of 2 eq. allyl isocyanate (1.55 g, 2.26 mmol, 2 eq., MM = 82.09 $g.mol^{-1}$) was added for each hydroxyl group. The solution was stirred for 1h at 60°C. $^1$H-NMR spectroscopy was used in order to control the progress of the transition of the hydroxyl functionalities to an alkene end group. Ultimately, the EUP-PCL product was purified through precipitation in cold diethyl ether, while fast stirring followed by filtration. The synthesis of tetra-functional EUP-PCL with a molar mass of 8000 $g.mol^{-1}$ proceeded similarly. Glycerol initiator was replaced by a pentaerythritol initiator (0.346 g, 2.534 mmol, 1: 69 stoichiometric ratio initiator to monomer, 136.15 $g.mol^{-1}$).

*Preparation of thiol-ene photo-crosslinked EUP-PCL sheets:*

A mixture of EUP-PCL (4 g, 2000 $g.mol^{-1}$), TPO-L as photo-initiator (10.4 mg, 2.6 wt% or 1.7mol% according to the EUP-PCL content) and thiol-based PETA 4SH crosslinker (0.438 g, 89.6 mmol, 488.66 g/mol) was dissolved in chloroform to homogenize the suspension. The PETA crosslinker was added according to an equimolar thiol to alkene ratio. The alkene content (8.96 $mmol.g^{-1}$) was determined with $^1$H-NMR spectroscopy with a DMT internal standard. Subsequently, the chloroform was evaporated completely and the remaining solution was poured between two glass plates separated by a silicone spacer (0.5 mm thickness). Finally, the mould was placed between UV lamps and was crosslinked into sheets upon UVA irradiation (365 nm, 10 $mW/cm^2$) for 30 minutes. Upon preparation of sheets with other molar masses, the amount of PETA 4SH crosslinker was altered according to the alkene content. Furthermore, sheets with PETA 3SH were synthesized similarly.

*Tensile testing:*

Tensile tests were conducted on an electromechanical universal 5ST tensile machine (Tinius Olsen) with a load cell of 500 N. Dogbone shapes were punched out from a cross-linked film. The samples had a gage length of 20.0 mm, a thickness of 0.5 mm and a width of 4.0 mm. The test continued at a crosshead speed of 10 mm/min and with a preload tension of 0.1 N until fracture of the specimen.

**References**

1. Tian, G. et al. The effects of PCL diol molecular weight on properties of shape memory poly(ε-caprolactone) networks. J. Appl. Polym. Sci. 136, 1-8 (2019).
2. Nagata, M. and Yamamoto, Y. (2009), Synthesis and characterization of photocrosslinked poly(ε-caprolactone)s showing shape-memory properties. J. Polym. Sci. A Polym. Chem., 47: 2422-2433. https://doi.org/10.1002/pola.23333.


**Claims**

**1.** A combination comprising:

- at least a polyester having a crystalline backbone and at least one end-capping group, wherein the end-capping group comprises any one of an ester, urethane, thiocarbamate or urea moiety connected either directly or by means of a spacer to a first crosslinkable group;
- at least a compound comprising a second crosslinkable group;

wherein the first and second crosslinkable groups are selected from either:

- a thiol comprising group; or
- an -ene group of formula (I),

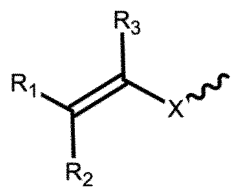

(I)

wherein

◦ $R_1$, $R_2$, $R_3$ are independently selected from: H, alkyl, O, N, halogen, S or at least one of $R_1$, $R_2$, $R_3$ together with the group at position X form a cyclic or heterocyclic structure;
◦ X is a group selected from: alkyl, O, N, S, (C=O)N, (C=O)alkyl, O-alkyl, N-alkyl, S-alkyl, and

wherein at least one group of the first and second crosslinkable groups is the thiol comprising group and at least one group of the first and second crosslinkable groups is the -ene group of formula (I), and
wherein the polyester and the compound comprise at least 2 of a first, respectively second crosslinkable groups, and the sum of said first, respectively second crosslinkable groups, is at least 5, and wherein the molar ratio of the first crosslinkable group to the second crosslinkable group is from 0.75 to 1.5, preferably from 0.8 to 1.2.

**2.** The combination according claim 1, wherein the polyester is of formula (B)

$$((A_m)_y - Y_m - Z_m)_n \text{ - backbone (B)}$$

wherein:

- $((A_m)_y - Y_m - Z_m)_n$ represents at least one end-capping group, and
- $A_m$ represents the first crosslinkable group;

- $Y_m$ is selected from the list comprising: the direct bond or the spacer;
- $Z_m$ represents the moiety selected from ester, urethane, thiocarbamate or urea, connected to said backbone, directly or indirectly; and

wherein n is integer and defines the number of arms/branches connected to the backbone, with $n \geq 1$, and wherein y is an integer and defines the number of first crosslinkable groups, and m is an enumerator for each arm/branch,

**3.** The combination according to any one of claims 1 to 2, wherein the end-capping group comprises any one of urethane, thiocarbamate or urea moiety.

**4.** The combination according to any one of claims 1 to 3, wherein:

- the end-capping group comprises a urethane moiety and the first crosslinkable group is the - ene group moiety of formula (I).

**5.** The combination according to claims 1 to 4, wherein the polyester comprises at least 2 end-capping groups comprising each at least one -ene group moiety of formula (I).

**6.** The combination according to any one of claims 1 to 5, wherein the backbone has a crystallinity in the range from about 10% to 80%, preferably from 20% to 70%, more preferably from 30% to 60%.

**7.** The combination according to any one of claims 1 to 6, wherein the backbone has a molar mass in the range from about 500 $g.mol^{-1}$ to 20000 $g.mol^{-1}$, preferably from 2000 $g.mol^{-1}$ to 10000 $g.mol^{-1}$.

**8.** The combination according to any one of claims 1 to 7, wherein the polyester has a geometry selected from: linear, star-shaped, preferably star-shaped.

**9.** The combination according any one of the previous claims, wherein the polyester backbone is selected from poly(ε-caprolactone) (PCL), poly(lactic acid) (PLA), poly(lactic-co-glycolic acid) (PLGA), polyglycolic acid (PGA) and copolymers thereof, preferably PLA and/or PCL.

**10.** The combination according to any one of the previous claims, wherein the -ene group of formula (I) is selected from: alkene, norbornene, allyl ether, vinyl ether, maleimide, preferably alkene, norbornene, allyl ether, vinyl ether.

**11.** The combination according to any one of the previous claims, wherein the compound comprising the second crosslinkable group is selected from: 1,2-Ethanedithiol, 1,3-Propanedithiol, 1,4-Butanedithiol, 2,2'-Thiodiethanethiol, 2,2'-(Ethylenedioxy)diethanethiol, Trimethylolpropane tris(3-mercaptopropionate), Pentaerythritol tetrakis(3-mercaptopropionate), 2-hydroxymethyl-2-methyl-1,3-propanediol tris-(3-mercaptopropionate) and combinations thereof.

**12.** A crosslinked polyester comprising a combination as defined in claims 1 to 11 wherein the polyester and the compound form a crosslinked network.

**13.** A process to produce a crosslinked polyester according to claim 12, comprising the steps of:

a) providing a combination as defined in anyone of claims 1 to 11,
b) crosslinking the combination provided at step a).

**14.** The process according to claim 13, wherein step a) further comprises providing an initiator to the combination, such as a photoinitiator, a thermal initiator or a redox initiator.

**15.** Use of a combination as defined in claims 1 to 11 and/or a crosslinked polyester as defined in claim 12, in additive manufacturing as a bioink, bioengineering, biomedical applications, such as implants, preferably biodegradable implants, such as bone implants, cartilage implants.

## Patentansprüche

**1.** Kombination, umfassend:

- mindestens einen Polyester, der ein kristallines Rückgrat und mindestens eine Endverkappungsgruppe aufweist, wobei die Endverkappungsgruppe einen beliebigen eines Ester-, Urethan-, Thiocarbamat- oder Harnstoffrests umfasst, der entweder direkt oder mittels eines Abstandshalters mit einer ersten vernetzbaren Gruppe verbunden ist;
- mindestens eine Verbindung, umfassend eine zweite vernetzbare Gruppe;

wobei die erste und die zweite vernetzbare Gruppe ausgewählt sind aus entweder:

- einer Thiol-umfassenden Gruppe; oder
- einer -en-Gruppe der Formel (I),

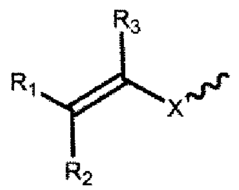

(I)

wobei

o $R_1$, $R_2$, $R_3$ unabhängig voneinander ausgewählt werden aus: H, Alkyl, O, N, Halogen, S oder mindestens eines von $R_1$, $R_2$, $R_3$ zusammen mit der Gruppe an Position X eine zyklische oder heterozyklische Struktur ausbilden;
o X eine Gruppe ist, die ausgewählt ist aus: Alkyl, O, N, S, (C=O)N, (C=O)Alkyl, O-Alkyl, N-Alkyl, S-Alkyl, und

wobei mindestens eine Gruppe der ersten und der zweiten vernetzbaren Gruppe die Thiol-umfassende Gruppe ist und mindestens eine Gruppe der ersten und der zweiten vernetzbaren Gruppen die -en-Gruppe der Formel (I) ist, und
wobei der Polyester und die Verbindung mindestens zwei einer ersten beziehungsweise einer zweiten vernetzbaren Gruppe umfassen und die Summe der ersten beziehungsweise der zweiten vernetzbaren Gruppe mindestens 5 beträgt, und wobei das Molverhältnis der ersten vernetzbaren Gruppe zu der zweiten vernetzbaren Gruppe von 0,75 bis 1,5, vorzugsweise von 0,8 bis 1,2 beträgt.

**2.** Kombination nach Anspruch 1, wobei der Polyester die Formel (B) hat

$((A_m)_y - Y_m - Z_m)_n$ -Rückgrat (B)

wobei:

- $((A_m)_y - Y_m - Z_m)_n$ mindestens eine Endverkappungsgruppe darstellt, und
- $A_m$ die erste vernetzbare Gruppe darstellt;
- $Y_m$ aus der Liste ausgewählt wird, umfassend: die direkte Bindung oder den Abstandshalter;
- $Z_m$ den Rest darstellt, der aus Ester, Urethan, Thiocarbamat oder Harnstoff ausgewählt ist, der direkt oder indirekt mit dem Rückgrat verbunden ist; und

wobei n eine ganze Zahl ist und die Anzahl der mit dem Rückgrat verbundenen Arme/Zweige definiert, wobei $n \geq 1$, und wobei y eine ganze Zahl ist und die Anzahl von ersten vernetzbaren Gruppen definiert, und m ein Zähler für jeden Arm/Zweig ist.

**3.** Kombination nach einem der Ansprüche 1 bis 2, wobei die Endverkappungsgruppe einen beliebigen eines Urethan-, Thiocarbamat- oder Harnstoffrests umfasst.

**4.** Kombination nach einem der Ansprüche 1 bis 3, wobei:

- die Endverkappungsgruppe einen Urethanrest umfasst und die erste vernetzbare Gruppe der -en-Gruppenrest der Formel (I) ist.

5. Kombination nach den Ansprüchen 1 bis 4, wobei der Polyester mindestens 2 Endverkappungsgruppen umfasst, jeweils umfassend mindestens einen -en-Gruppenrest der Formel (I).

6. Kombination nach einem der Ansprüche 1 bis 5, wobei das Rückgrat eine Kristallinität in dem Bereich von etwa 10 % bis 80 %, vorzugsweise von 20 % bis 70 %, mehr bevorzugt von 30 % bis 60 % aufweist.

7. Kombination nach einem der Ansprüche 1 bis 6, wobei das Rückgrat eine Molmasse in dem Bereich von etwa 500 $g.mol^{-1}$ bis 20000 $g.mol^{-1}$, vorzugsweise von 2000 $g.mol^{-1}$ bis 10000 $g.mol^{-1}$ aufweist.

8. Kombination nach einem der Ansprüche 1 bis 7, wobei der Polyester eine Geometrie aufweist, die ausgewählt ist aus: linear, sternförmig, vorzugsweise sternförmig.

9. Kombination nach einem der vorstehenden Ansprüche, wobei das Polyester-Rückgrat aus Poly(ε-Caprolacton) (PCL), Poly(milchsäure) (PLA), Poly(milch-co-glykolsäure) (PLGA), Polyglykolsäure (PGA) und Copolymeren davon, vorzugsweise PLA und/oder PCL, ausgewählt ist.

10. Kombination nach einem der vorstehenden Ansprüche, wobei die -en-Gruppe der Formel (I) ausgewählt ist aus: Alken, Norbornen, Allylether, Vinylether, Maleimid, vorzugsweise Alken, Norbornen, Allylether, Vinylether.

11. Kombination nach einem der vorstehenden Ansprüche, wobei die Verbindung, umfassend die zweite vernetzbare Gruppe, ausgewählt ist aus: 1,2-Ethandithiol, 1,3-Propandithiol, 1,4-Butandithiol, 2,2'-Thiodiethanthiol, 2,2'-(Ethylendioxy)diethanthiol, Trimethylolpropantris(3-mercaptopropionat), Pentaerythrittetrakis(3-mercaptopropionat), 2-Hydroxymethyl-2-methyl-1,3-propandioltris-(3-mercaptopropionat) und Kombinationen davon.

12. Vernetzter Polyester, umfassend eine Kombination nach den Ansprüchen 1 bis 11, wobei der Polyester und die Verbindung ein vernetztes Netzwerk ausbilden.

13. Verfahren, um einen vernetzten Polyester nach Anspruch 12 herzustellen, umfassend die Schritte:

    a) Bereitstellen einer Kombination nach einem der Ansprüche 1 bis 11,
    b) Vernetzen der in Schritt a) bereitgestellten Kombination.

14. Verfahren nach Anspruch 13, wobei Schritt a) ferner das Bereitstellen eines Initiators an die Kombination, wie etwa eines Photoinitiators, eines thermischen Initiators oder eines Redoxinitiators, umfasst.

15. Verwendung einer Kombination nach den Ansprüchen 1 bis 11 und/oder eines vernetzten Polyesters nach Anspruch 12 in einer additiven Fertigung als eine Biotinte, in der Biotechnik, in biomedizinischen Anwendungen, wie etwa Implantaten, vorzugsweise biologisch abbaubaren Implantaten, wie etwa Knochenimplantaten, Knorpelimplantaten.

## Revendications

1. Combinaison comprenant :

    - au moins un polyester ayant un squelette cristallin et au moins un groupe de coiffage terminal, dans laquelle le groupe de coiffage terminal comprend l'un quelconque parmi un fragment ester, uréthane, thiocarbamate ou urée relié soit directement soit au moyen d'un espaceur à un premier groupe réticulable ;
    - au moins un composé comprenant un second groupe réticulable ;
    dans laquelle les premier et second groupes réticulables sont choisis parmi soit :

        - un groupe comprenant un thiol ; soit
        - un groupe -ène de formule (I),

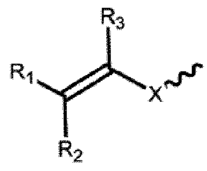

(I)

dans laquelle

- ◦ $R_1$, $R_2$, $R_3$ sont indépendamment choisis parmi : H, alkyle, O, N, halogène, S ou au moins l'un parmi $R_1$, $R_2$, $R_3$ conjointement avec le groupe à la position X forme une structure cyclique ou hétérocyclique ;
- ◦ X est un groupe choisi parmi : alkyle, O, N, S, (C=O)N, (C=O)alkyle, O-alkyle, N-alkyle, S-alkyle, et

dans laquelle au moins un groupe des premier et second groupes réticulables est le groupe comprenant un thiol et au moins un groupe des premier et second groupes réticulables est le groupe -ène de formule (I), et dans laquelle le polyester et le composé comprennent au moins 2 de premiers, et respectivement seconds groupes réticulables, et la somme desdits premiers, et respectivement seconds groupes réticulables, vaut au moins 5, et dans laquelle le rapport molaire du premier groupe réticulable au second groupe réticulable va de 0,75 à 1,5, de préférence de 0,8 à 1,2.

**2.** Combinaison selon la revendication 1, dans laquelle le polyester est de formule (B)

$((A_m)_y\text{-}Y_m - Z_m)_n$ -squelette (B)

dans laquelle :

- $((A_m)_y - Y_m - Z_m)_n$ représente au moins un groupe de coiffage terminal, et
- $A_m$ représente le premier groupe réticulable ;
- $Y_m$ est choisi parmi la liste comprenant : la liaison directe ou l'espaceur ;
- $Z_m$ représente le fragment choisi parmi ester, uréthane, thiocarbamate ou urée, relié audit squelette, directement ou indirectement ; et

dans laquelle n est un nombre entier et définit le nombre de bras/ramifications reliés au squelette, avec $n \geq 1$, et dans laquelle y est un nombre entier et définit le nombre de premiers groupes réticulables, et m est un énumérateur pour chaque bras/ramification.

**3.** Combinaison selon l'une quelconque des revendications 1 à 2, dans laquelle le groupe de coiffage terminal comprend l'un quelconque parmi un fragment uréthane, thiocarbamate ou urée

**4.** Combinaison selon l'une quelconque des revendications 1 à 3, dans laquelle :

- le groupe de coiffage terminal comprend un fragment uréthane et le premier groupe réticulable est le fragment de groupe -ène de formule (I).

**5.** Combinaison selon les revendications 1 à 4, dans laquelle le polyester comprend au moins 2 groupes de coiffage terminal comprenant chacun au moins un fragment de groupe -ène de formule (I).

**6.** Combinaison selon l'une quelconque des revendications 1 à 5, dans laquelle le squelette a une cristallinité dans la plage allant d'environ 10 % à 80 %, de préférence de 20 % à 70 %, plus préférablement de 30 % à 60 %.

**7.** Combinaison selon l'une quelconque des revendications 1 à 6, dans laquelle le squelette a une masse molaire dans la plage allant d'environ 500 $g.mol^{-1}$ à 20 000 $g.mol^{-1}$, de préférence de 2000 $g.mol^{-1}$ à 10 000 $g.mol^{-1}$.

**8.** Combinaison selon l'une quelconque des revendications 1 à 7, dans laquelle le polyester a une géométrie choisie parmi : linéaire, en forme d'étoile, de préférence en forme d'étoile.

**9.** Combinaison selon l'une quelconque des revendications précédentes, dans laquelle le squelette polyester est choisi parmi poly(ε-caprolactone) (PCL), poly(acide lactique) (PLA), acide poly(lactique-co-glycolique) (PLGA), acide

polyglycolique (PGA) et copolymères de ceux-ci, de préférence PLA et/ou PCL.

**10.** Combinaison selon l'une quelconque des revendications précédentes, dans laquelle le groupe -ène de formule (I) est choisi parmi : alcène, norbornène, éther allylique, éther vinylique, maléimide, de préférence alcène, norbornène, éther allylique, éther vinylique.

**11.** Combinaison selon l'une quelconque des revendications précédentes, dans laquelle le composé comprenant le second groupe réticulable est choisi parmi : 1,2-éthanedithiol, 1,3-propanedithiol, 1,4-butanedithiol, 2,2'-thiodiethanethiol, 2,2'-(éthylènedioxy)diéthanethiol, tris(3-mercaptopropionate) de triméthylolpropane, tétrakis(3-mercaptopropionate) de pentaérythritol, tris-(3-mercaptopropionate) de 2-hydroxyméthyl-2-méthyl-1,3-propanediol et combinaisons de ceux-ci.

**12.** Polyester réticulé comprenant une combinaison selon les revendications 1 à 11 dans lequel le polyester et le composé forment un réseau réticulé.

**13.** Procédé pour produire un polyester réticulé selon la revendication 12, comprenant les étapes consistant à :

a) fournir une combinaison selon l'une quelconque des revendications 1 à 11,
b) réticuler la combinaison fournie à l'étape a).

**14.** Procédé selon la revendication 13, dans lequel l'étape a) comprend en outre la fourniture d'un initiateur à la combinaison, tel qu'un photoinitiateur, un initiateur thermique ou un initiateur redox.

**15.** Utilisation d'une combinaison selon les revendications 1 à 11 et/ou d'un polyester réticulé selon la revendication 12, en fabrication additive en tant que bio-encre, en bio-ingénierie, dans des applications biomédicales, telles que des implants, de préférence des implants biodégradables, tels que des implants osseux, des implants cartilagineux.

**Fig. 1**

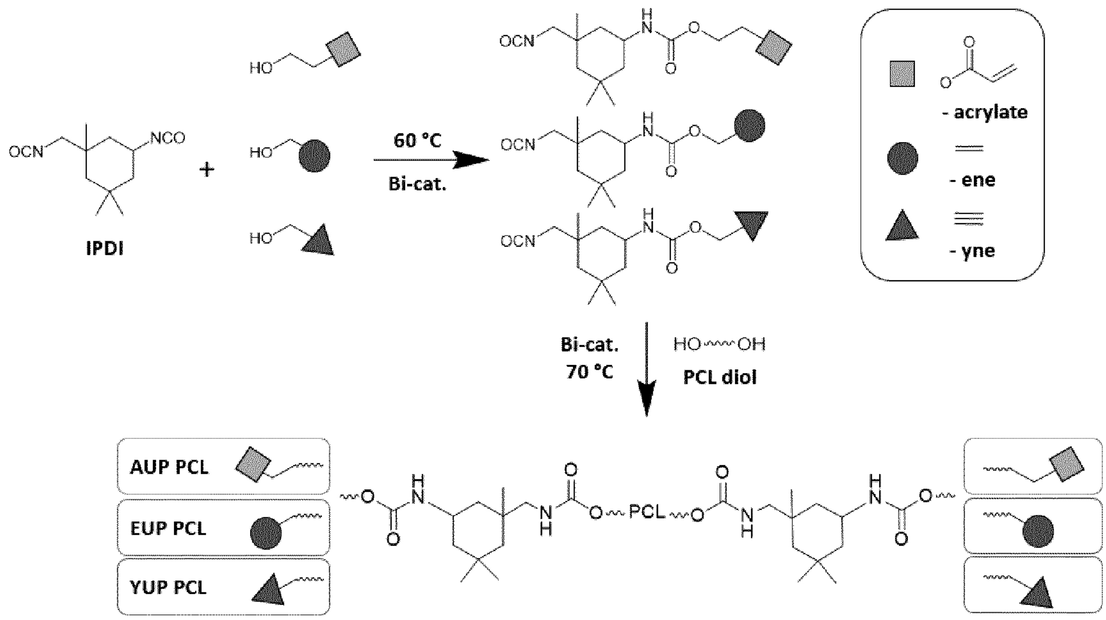
IPDI
60 °C
Bi-cat.
- acrylate
- ene
- yne
Bi-cat.
70 °C
PCL diol
AUP PCL
EUP PCL
YUP PCL

**Fig. 2**

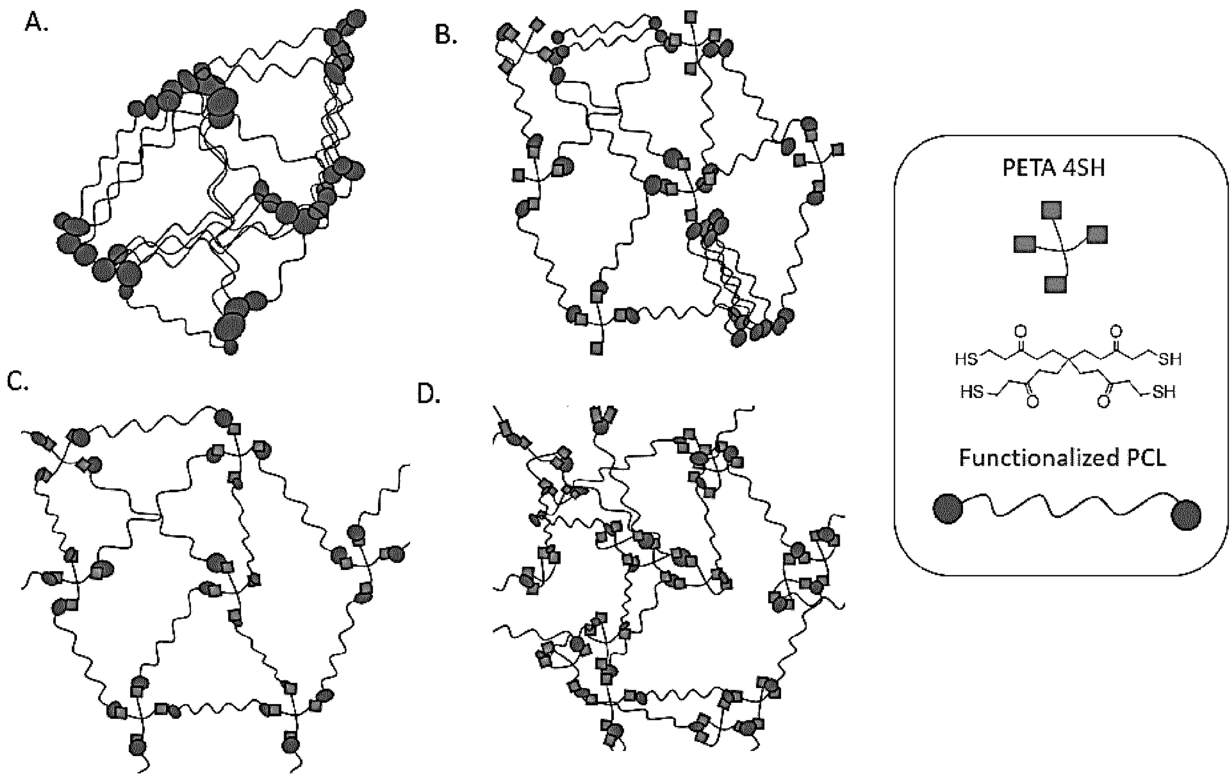
A.
B.
C.
D.
PETA 4SH
Functionalized PCL

Fig. 3

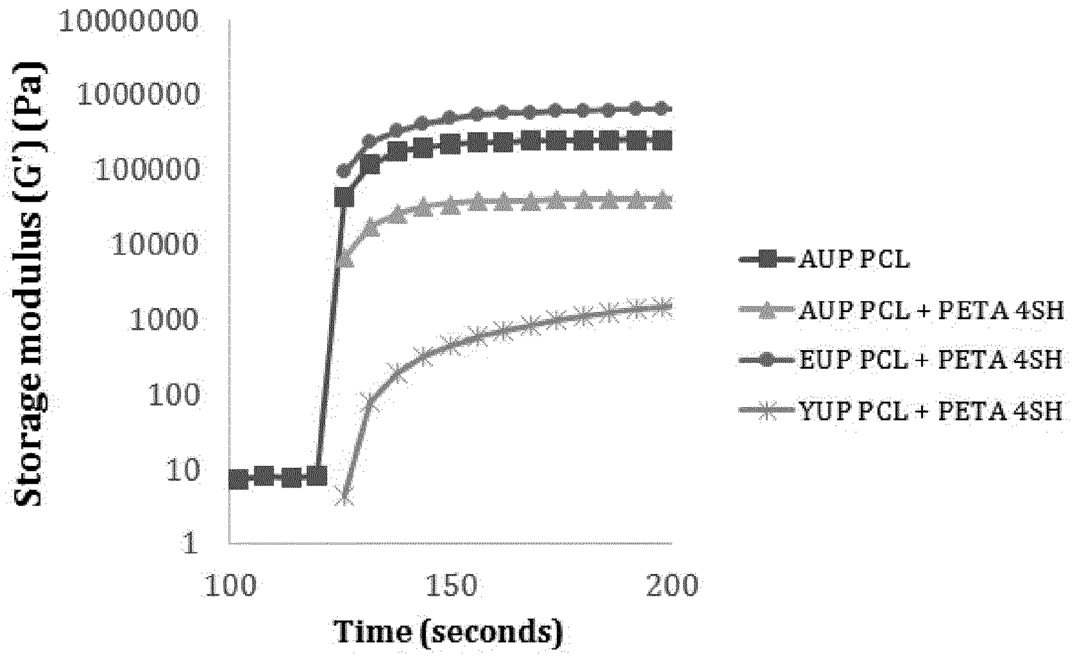
Storage modulus (G') (Pa)
10000000
1000000
100000
10000
1000
100
10
1
100
150
200
Time (seconds)
AUP PCL
AUP PCL + PETA 4SH
EUP PCL + PETA 4SH
YUP PCL + PETA 4SH

Fig. 4

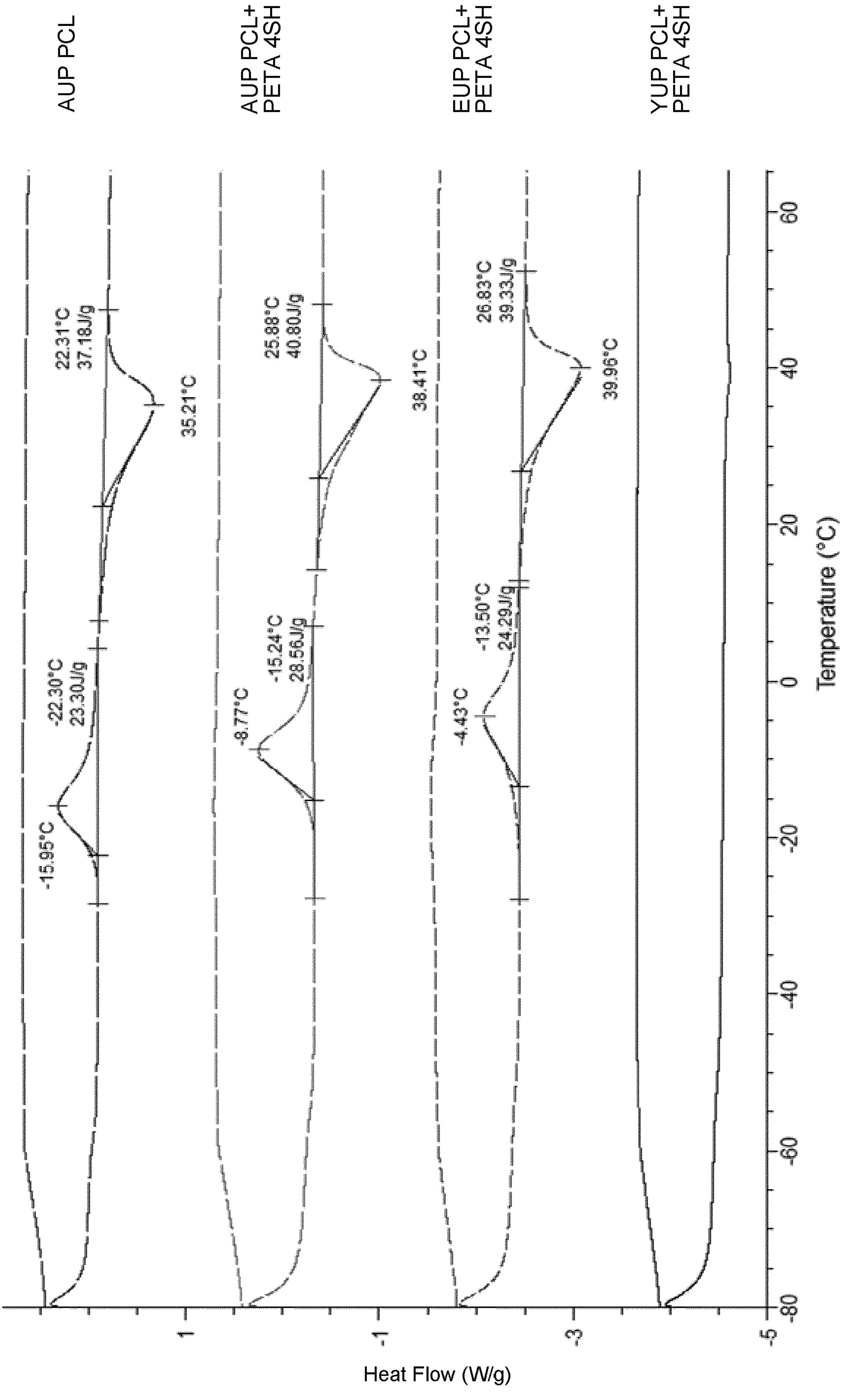
AUP PCL
AUP PCL+ PETA 4SH
EUP PCL+ PETA 4SH
YUP PCL+ PETA 4SH
22.31°C
37.18J/g
35.21°C
-22.30°C
23.30J/g
-15.95°C
25.88°C
40.80J/g
38.41°C
-15.24°C
28.56J/g
-8.77°C
26.83°C
39.33J/g
39.96°C
-13.50°C
24.29J/g
-4.43°C
Temperature (°C)
Heat Flow (W/g)
60
40
20
0
-20
-40
-60
-80
1
-1
-3
-5

Fig. 5

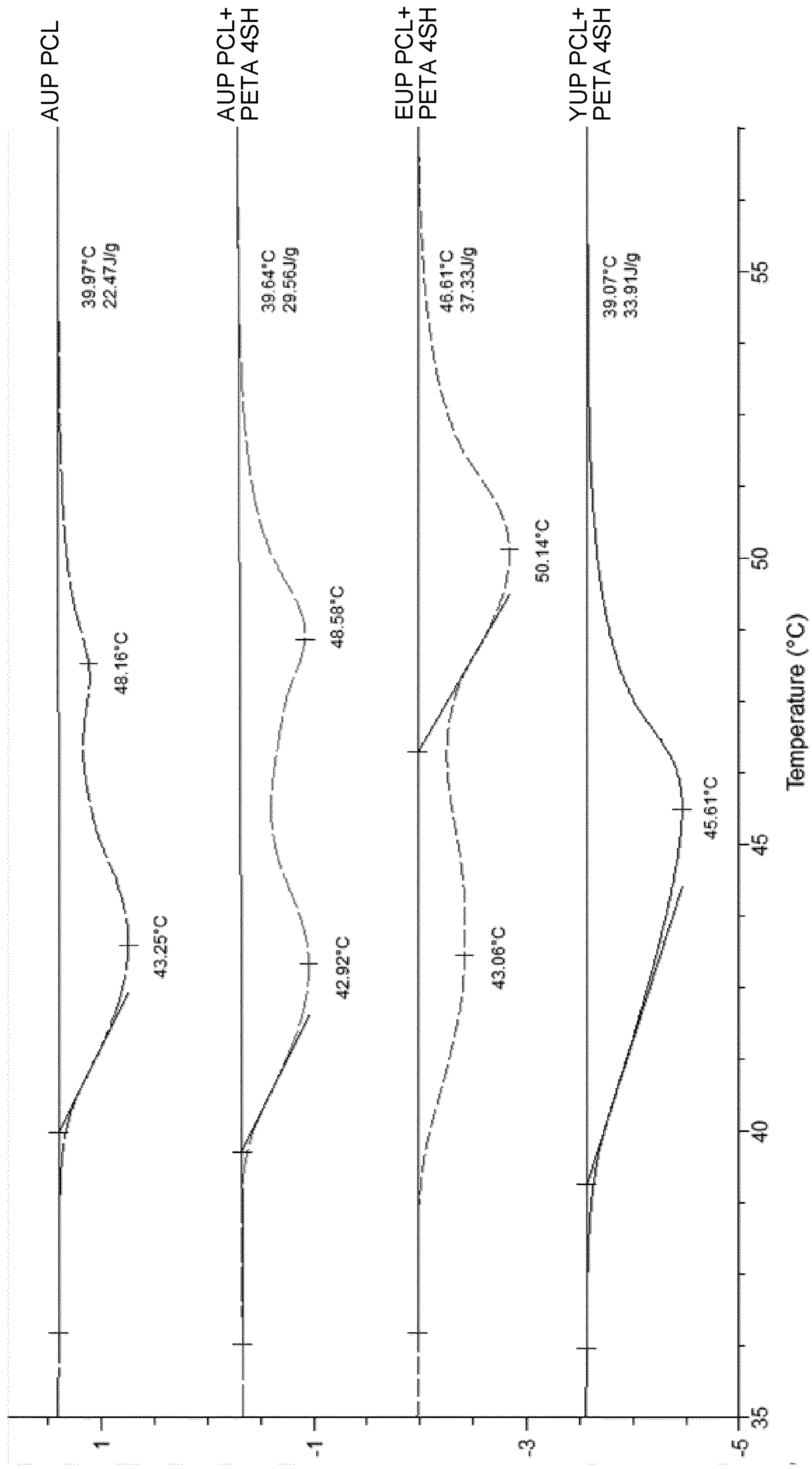
AUP PCL
AUP PCL+ PETA 4SH
EUP PCL+ PETA 4SH
YUP PCL+ PETA 4SH
39.97°C
22.47J/g
48.16°C
43.25°C
39.64°C
29.56J/g
48.58°C
42.92°C
46.61°C
37.33J/g
50.14°C
43.06°C
39.07°C
33.91J/g
45.61°C
35
40
45
50
55
Temperature (°C)
1
-1
-3
-5
Heat Flow (W/g)

Fig. 6

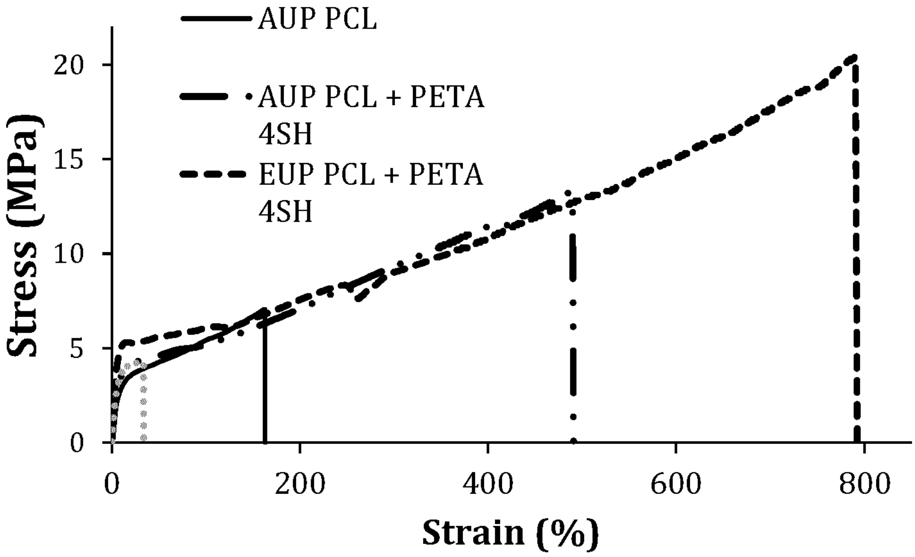
AUP PCL
AUP PCL + PETA 4SH
EUP PCL + PETA 4SH
Stress (MPa)
0
5
10
15
20
0
200
400
600
800
Strain (%)

Fig. 7

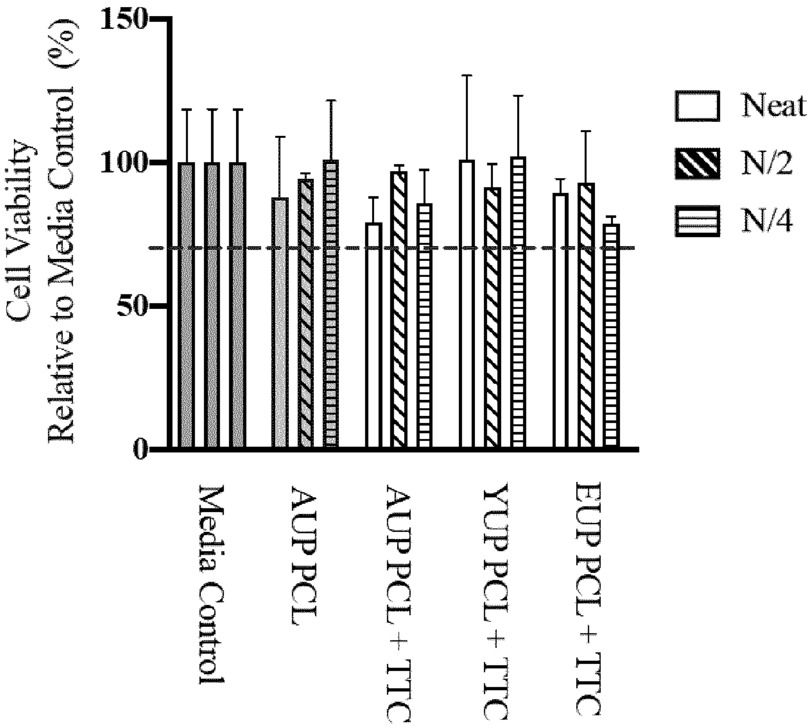
Cell Viability
Relative to Media Control (%)
0
50
100
150
Neat
N/2
N/4
Media Control
AUP PCL
AUP PCL + TTC
YUP PCL + TTC
EUP PCL + TTC

Fig. 8

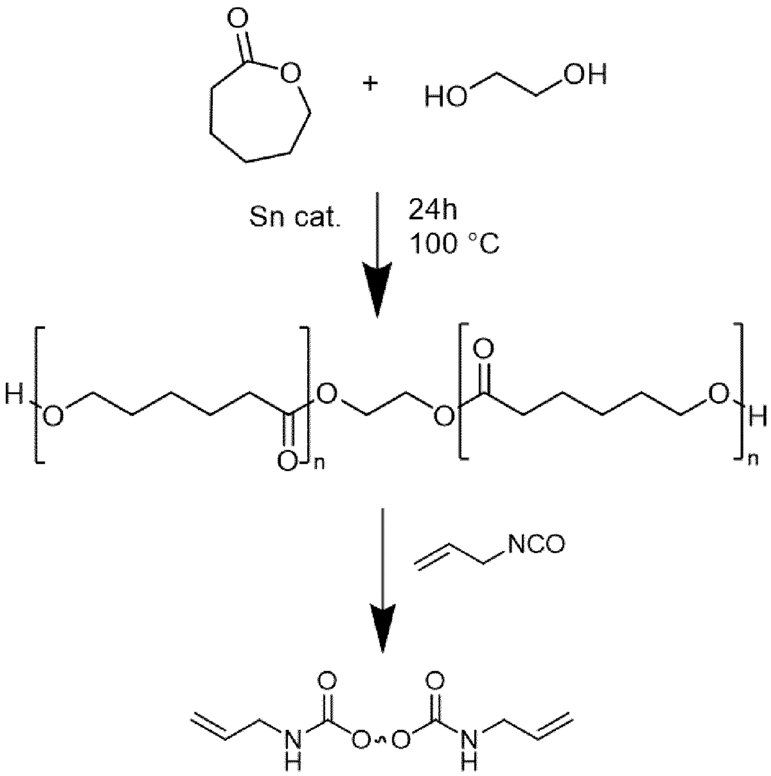
Sn cat.
24h
100 °C

Fig. 9

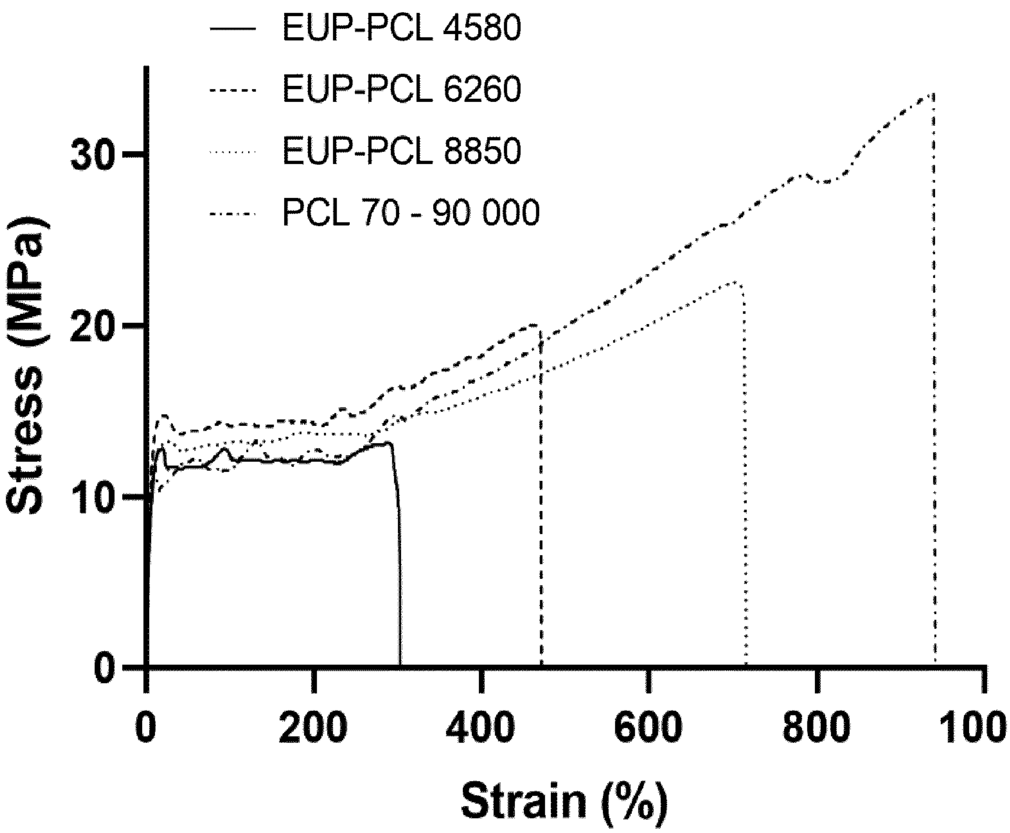
EUP-PCL 4580
EUP-PCL 6260
EUP-PCL 8850
PCL 70 - 90 000
Stress (MPa)
0
10
20
30
0
200
400
600
800
100
Strain (%)

Fig. 10

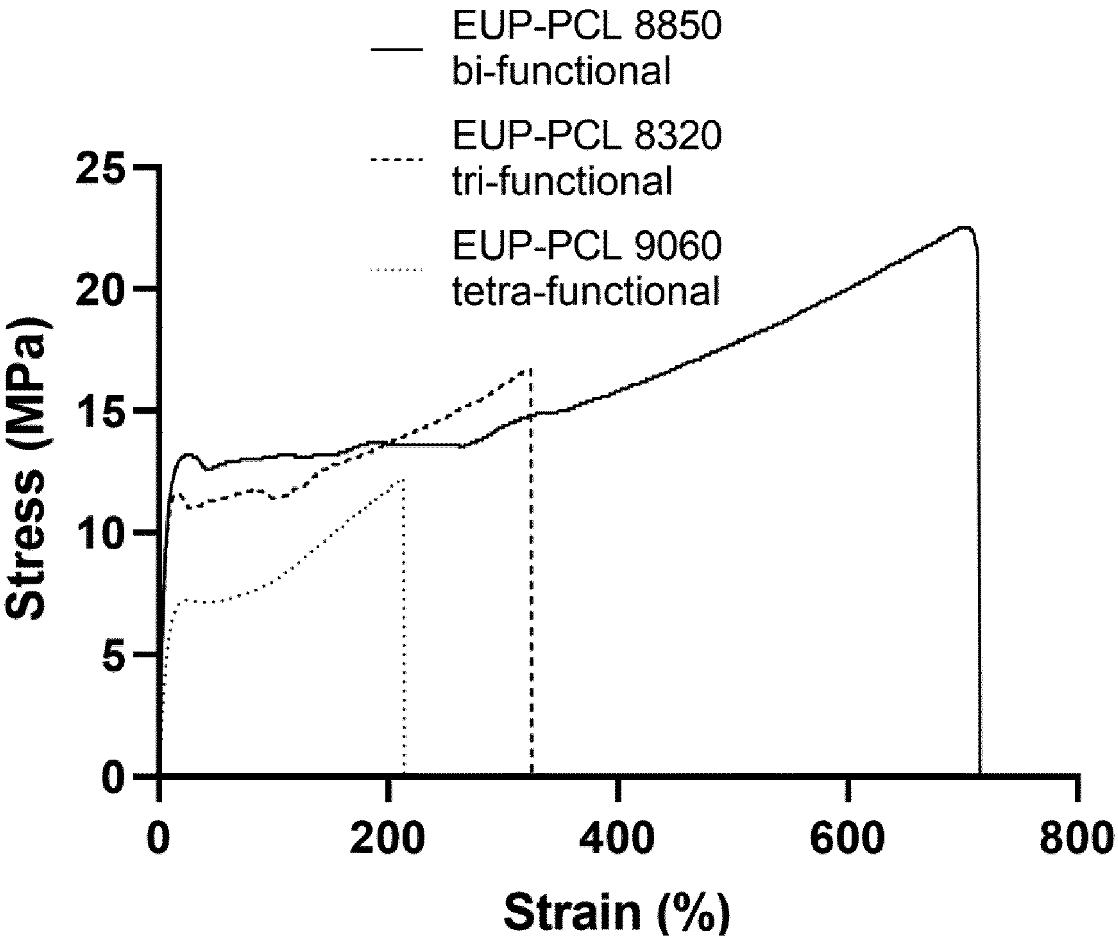
EUP-PCL 8850
bi-functional
EUP-PCL 8320
tri-functional
EUP-PCL 9060
tetra-functional
Stress (MPa)
25
20
15
10
5
0
0
200
400
600
800
Strain (%)

Fig. 11

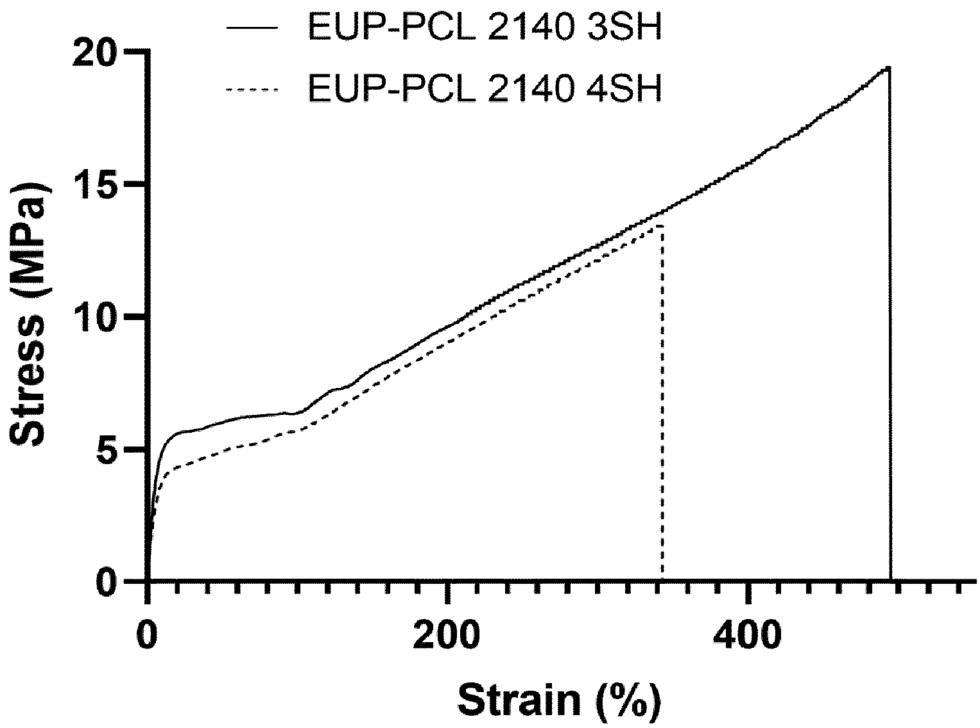
EUP-PCL 2140 3SH
EUP-PCL 2140 4SH
Stress (MPa)
20
15
10
5
0
0
200
400
Strain (%)

Fig. 12

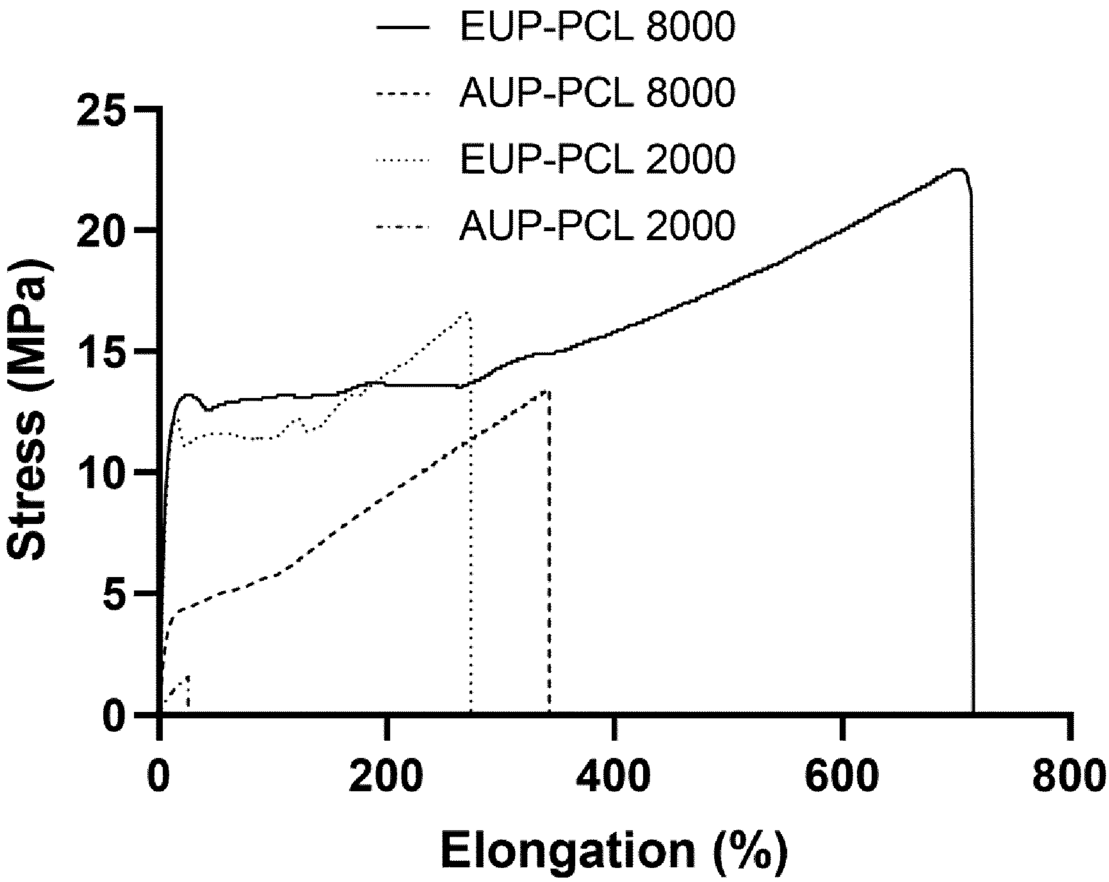
EUP-PCL 8000
AUP-PCL 8000
EUP-PCL 2000
AUP-PCL 2000
Stress (MPa)
25
20
15
10
5
0
0
200
400
600
800
Elongation (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020094621 A1


### Non-patent literature cited in the description


- **TIAN, G. et al.** The effects of PCL diol molecular weight on properties of shape memory poly(ε-caprolactone) networks.. *J. Appl. Polym. Sci.*, 2019, vol. 136, 1-8
- **NAGATA, M.** ; **YAMAMOTO, Y.** Synthesis and characterization of photocrosslinked poly(ε-caprolactone)s showing shape-memory properties. *J. Polym. Sci. A Polym. Chem.*, 2009, vol. 47, 2422-2433, https://doi.org/10.1002/pola.23333